(19) Europäisches Patentamt
European Patent Office
Office européen des brevets

(11) **EP 1 524 317 B1**

(12) **EUROPEAN PATENT SPECIFICATION**

(45) Date of publication and mention
of the grant of the patent:
**04.03.2015 Bulletin 2015/10**

(51) Int Cl.:
***C12N 15/10*** *(2006.01)*

(21) Application number: **04024138.2**

(22) Date of filing: **09.10.2004**

(54) **Methods for isolating nucleic acids**

Verfahren zur Isolierung von Nukleinsäuren

Procédé pour l'isolation d'acides nucléiques

(84) Designated Contracting States:
**AT BE BG CH CY CZ DE DK EE ES FI FR GB GR
HU IE IT LI LU MC NL PL PT RO SE SI SK TR**

(30) Priority: **13.10.2003 EP 03023039
28.07.2004 EP 04017856**

(43) Date of publication of application:
**20.04.2005 Bulletin 2005/16**

(73) Proprietors:
• **Roche Diagnostics GmbH
68305 Mannheim (DE)**
Designated Contracting States:
**DE**
• **F. Hoffmann-La Roche AG
4070 Basel (CH)**
Designated Contracting States:
**AT BE BG CH CY CZ DK EE ES FI FR GB GR HU
IE IT LI LU MC NL PL PT RO SE SI SK TR**

(72) Inventors:
• **Bergmann, Frank, Dr.
82393 Iffeldorf (DE)**
• **Kirchgesser, Michael, Dr.
81379 Muenchen (DE)**
• **Walter, Thomas, Dr.
82377 Penzberg (DE)**
• **Weindel, Kurt, Dr.
82407 Wielenbach-Hardt (DE)**
• **Zielenski, Ralf
6006 Luzern (CH)**
• **Sarofim, Emad
6332 Hagendorn (CH)**

(74) Representative: **Merkel, Patrick et al
Roche Diagnostics International AG
Forrenstrasse 2
6343 Rotkreuz (CH)**

(56) References cited:
**EP-A- 0 512 767     EP-A- 0 757 106
WO-A1-01/37291     US-A- 5 124 444
US-A- 5 804 684     US-A1- 2001 041 332**

**Description**

The present invention is directed to the purification of a nucleic acid. Particularly, the invention is directed to methods of adsorbing a nucleic acid present in an aequous adsorption solution to a solid substrate.

Many biological substances, especially nucleic acids, present special challenges in terms of isolating them from their natural environment. On the one hand, they are often present in very small concentrations and, on the other hand, they are often found in the presence of many other solid and dissolved substances e.g. after lysis of cells. This makes them difficult to isolate or to measure, in particular in biospecific assays which allow the detection of specific nucleic acids, or the detection of specific properties of a nucleic acid. Such biospecific assays play a major role in the field of diagnostics and bioanalytics in research and development. Examples for biospecific assays are hybridisation assays, immuno assays and receptor-ligand assays. Hybridisation assays use the specific base-pairing for the molecular detection of nucleic acid analytes e.g. RNA and DNA. Hence, oligonucleotide probes with a length of 18 to 20 nucleotides may enable the specific recognition of a selected complementary sequence e.g. in the human genome. Another assay which entails the selective binding of two oligonucleotide primers is the polymerase chain reaction (PCR) described in US 4,683,195. This method allows the selective amplification of a specific nucleic acid region to detectable levels by a thermostable polymerase in the presence of desoxynucleotide triphosphates in several cycles.

As described above, before the nucleic acids may be analyzed in one of the above-mentioned assays or used for other processes, they have to be isolated or purified from biological samples containing complex mixtures of different components as e.g. proteinaceous and non-proteinaceous components. Often, for the first steps, processes are used which allow the enrichment of the component of interest, i.e. the nucleic acids. Frequently, these are contained in a bacterial cell, a fungal cell, a viral particle, or the cell of a more complex organism, such as a human blood cell or a plant cell. Nucleic acids as a component of interest can also be called a "target component".

To release the contents of said cells or particles, they may be treated with enzymes or with chemicals to dissolve, degrade or denature the cellular walls and cellular membranes of such organisms. This process is commonly referred to as lysis. The resulting solution containing such lysed material is referred to as lysate. A problem often encountered during the lysis is that other enzymes degrading the target component, e.g. desoxyribonucleases or ribonucleases degrading nucleic acids, come into contact with the target component during lysis. These degrading enzymes may also be present outside the cells or may have been spatially separated in different cellular compartments before the lysis and come now into contact with the target component. Other components released during this process may be e.g. endotoxins belonging to the family of lipopolysaccharides which are toxic to cells and can cause problems for products intended to be used in human or animal therapy.

In the next steps of the sample preparation which follow on the lysis step, the nucleic acids are further enriched. Nucleic acids are normally extracted from the complex lysis mixtures before they are used in a probe-based assay. There are several methods for the extraction of nucleic acids. Sequence-dependent or biospecific methods include, e.g., affinity chromatography or hybridisation to immobilised probes. Sequence-independent or physico-chemical methods include, e.g., liquid-liquid extraction with phenol-chloroform, precipitation with pure ethanol or isopropanol, extraction with filter paper, extraction with micelle-forming agents as cetyl-trimethyl-ammonium-bromide, binding to immobilized, intercalating dyes such as acridine derivatives, adsorption to substrates such as silica gel or diatomic earths, adsorption to magnetically attractable glass particles (MGP) or organo silane particles under chaotropic conditions. Direct binding of the nucleic acids to a substrate such as a material with a silica surface is preferred because among other reasons the nucleic acids do not have to be modified and even native nucleic acids can be bound.

Particularly interesting for extraction purposes is the adsorption of nucleic acids to a glass surface although other surfaces are possible.

Many procedures for isolating nucleic acids from their natural environment have been proposed in recent years by the use of their binding behavior to substrates such as glass surfaces. It is common to use chaotropic agents such as, e.g., guanidine thiocyanate or anionic, cationic, zwitterionic or non-ionic detergents when nucleic acids are intended to be set free. It is also an advantage to use proteases which rapidly degrade these enzymes or unwanted proteins. Nucleic acids which are set free, e.g. after cell lysis and/ or lysis of cellular organelles such as mitochondria, plastids, nuclei or other nucleic acid-containing organelles, can be purified by way of binding to a substrate such as a mineral support, washing said mineral support with the bound nucleic acids and releasing, i.e. desorbing said nucleic acids from said mineral support. For a washing step conditions are chosen by the skilled artisan, under which the nucleic acids remain adsorbed to the mineral support. Preferably, greater than 40%, more preferred greater than 50%, more preferred greater than 70%, more preferred greater than 80%, even more preferred greater than 90%, even more preferred greater than 95%, even more preferred greater than 99% of the nucleic acids remain adsorbed to the mineral support. For the desorbing step conditions are chosen by the skilled artisan, under which the nucleic acids are released from the mineral support. Preferably, greater than 40%, more preferred greater than 50%, more preferred greater than 70%, more preferred greater than 80%, even more preferred greater than 90%, even more preferred greater than 95%, even more preferred greater than 99% of the nucleic acids are released from the mineral support.

Adsorption of nucleic acids to glass particles or silica particles in the presence of chaotropic salts is known to the art (Vogelstein, B., and Gillespie, D., Proc. Natl. Acad. Sci. USA 76 (1979) 615-619) and provide the basis for chromatographic purification and separation processes for nucleic acids. Also known to the art are methods to isolate and purify RNA and DNA from lysates using high concentrations of chaotropic salts, e.g. sodium iodide, sodium perchlorate and guanidine thiocyanate (Boom, R., et al., J. Clin. Microbiol. 28 (1990) 495-503; Yamada, O., et al., J. Virol. Methods 27 (1990) 203-209). The purification of plasmid DNA from bacteria on glass dust in the presence of sodium perchlorate is described in Marko, M.A., et al., Anal. Biochem. 121 (1982) 382-387. In DE 37 24 442, the isolation of single-stranded M13 phage DNA on glass fiber filters by precipitating phage particles using acetic acid and lysis of the phage particles with perchlorate is described. The nucleic acids bound to the glass fiber filters are washed and then eluted with a methanol-containing Tris/EDTA buffer. A similar procedure for purifying DNA from lambda phages is described in Jakobi, R., et al., Anal. Biochem. 175 (1988) 196-201. The procedure entails the selective binding of nucleic acids to glass surfaces in chaotropic salt solutions and separating the nucleic acids from contaminants such as agarose, proteins or cell residue. To separate the glass particles from the contaminants, the particles may be either centrifuged or fluids are drawn through glass fiber filters. This is a limiting step, however, that prevents the procedure from being used to process large quantities of samples.

The use of magnetic particles to immobilize nucleic acids after precipitation by adding salt and ethanol is more advantageous and described e.g. in: Alderton, R.P., et al., Anal. Biochem. 201 (1992) 166-169 and WO 91/00212. In this procedure, the nucleic acids are agglutinated along with the magnetic particles. The agglutinate is separated from the original solvent by applying a magnetic field and performing a wash step. After one wash step, the nucleic acids are dissolved in a Tris buffer. This procedure has a disadvantage, however, in that the precipitation is not selective for nucleic acids. Rather, a variety of solid and dissolved substances are agglutinated as well. As a result, this procedure can not be used to remove significant quantities of any inhibitors of specific enzymatic reactions that may be present. Magnetic, porous glass is also available on the market that contains magnetic particles in a porous, particular glass matrix and is covered with a layer containing streptavidin. This product can be used to isolate biological materials, e.g., proteins or nucleic acids, if they are modified in a complex preparation step so that they bind covalently to biotin. Magnetizable particular adsorbents proved to be very efficient and suitable for automatic sample preparation. Ferrimagnetic and ferromagnetic as well as superparamagnetic pigments are used for this purpose. The most preferred MGPs are those described in WO 01/37291.

Purification of a nucleic acid by way of adsorbing the same to a substrate such as a mineral substrate in the presence of high concentration of salts is also applied to other complex mixtures. Examples therefor are known to the person skilled in the art of molecular biology and include reaction mixtures following, e.g., in-vitro synthesis of nucleic acids such as PCR, restriction enzyme digestions, ligation reactions, etc.. In Vogelstein, B., and Gillespie, D., Proc. Natl. Acad. Sci. USA 76 (1979) 615-619, for instance, a procedure for binding nucleic acids from agarose gels in the presence of sodium iodide to ground flint glass is proposed. Another application for purification of a nucleic acid by way of adsorbing the same to a substrate such as a mineral substrate in the presence of a high concentration of salts is the removal of pyrogenic contaminants which may have copurified with the nucleic acid.

The mechanism by which nucleic acids bind to the mineral support in the presence of chaotropic agents is not entirely clear. It is hypothesized that the interaction between the nucleic acids and the solvent is influenced such that the nucleic acids adsorb to the mineral support and denaturate. In the presence of high concentrations of chaotropic agents the reaction is almost quantitative. The adsorbed nucleic acids can be eluted by applying to the mineral support buffers of low ionic strength.

EP 0 658 164 describes a method for the chromatographic purification of nucleic acids by way of chromatographic purification. Nucleic acids are adsorbed to a substrate, i.e. a mineral support, from an aequous adsorption solution with a high salt concentration which preferably contains a chaotropic agent. The aequous adsorption solution comprises 1%-50% of aliphatic alcohol with a chain length of C1-C5 and/ or polyethylene glycol and/ or hydrophobic inorganic and/ or organic polymers and/ or organic acid such as trichloroacetic acid.

EP 0 512 767 describes methods for DNA purification with solid supports using binding buffers with organic compounds like acetonitrile in certain concentrations.

The methods for the isolation/purification of nucleic acids of the state of the art have certain disadvantages. Such disadvantages relate to e.g. purity, selectivty, recovery rate, laboratory safety and convenience, as well as to the speed of the isolation/purification process.

E.g., in protocols using a phenol/chloroform extraction, residual phenol is often a problem for certain post isolation procedures, particularly for enzymatic reactions such as a digestion with a restriction enzyme, the polymerase chain reaction (PCR), or a ligase-mediated reaction. Generally, elevated concentrations of residual reagents from the purification/isolation process may pose a problem. It is therefore desired to keep residual amounts of the reagents used during the purification procedure as low as possible in the purified nucleic acid. Another potential problem related to purity is the coextraction of certain substances from the adsorption matrix (leaching). It is therefore desired to keep residual amounts of compounds liberated during the purification procedure by leaching as low as possible in the purified nucleic acid.

Another disadvantage of state of the art protocols which use ethanol or isopropanol in the adorption solution is the high volatility and flammablity of such alcohols. On the one hand, these flammable alcohols are potential hazards in laboratory practice. Also, depending on national regulations, flammable alcohols may pose logistical problems with regard to allowable storage and transport. In addition, volatile alcohols are difficult to dose with precision because of their vapor pressure. It is therefore desired to replace flammable alcohols by substances which are less hazardous or/ and which pose less logistical problems.

Exemplary kits which are commercially available for sample preparation of nucleic acids are the "High Pure" product line (Roche Diagnostics GmbH, Mannheim, Germany). The adsorption solution is transferred to a "High Pure" column and passed through a fleece containing glass fiber material. During this process the nucleic acids are adsorbed to the glass material. When using the columns of the Roche "High Pure" kit and a protocol for nucleic acid isolation/purification from serum making use of ethanol in the adsorption solution it was noted that high triglyceride concentrations in serum lead to a prolonged time needed to pass the adsorption solution through the glass fiber fleece (also see Example 6). It is therefore desired to identify a substitute for ethanol which, considering sample preparation from serum with high triglyceride concentrations, reduces the time needed to pass the adsorption solution through the glass fiber fleece.

The problem underlying the present invention is therefore to provide an alternative method for the purification of a nucleic acid using alternative substances in the aequous adsorption solution, in order to facilitate the binding of a nucleic acid to a substrate such as a mineral support.

Surprisingly, it has been found that a nucleic acid can be bound to a substrate if the adsorption solution of high ionic strength contains a water-miscible non-acidic organic compound comprising a functional group of Formula I,

$$W \equiv Z \qquad \qquad \text{(Formula I)}$$

whereby W is a carbon atom or a sulfur atom and Z is an oxygen atom or a nitrogen atom.

Thus, a useful method for the purification of a nucleic acid comprises the steps of: a) adsorbing on a substrate the nucleic acid from a composition containing (i) an aequous buffer, (ii) salts in a high concentration and (iii) a water-miscible non-acidic organic compound comprising a functional group of Formula I,

$$W \equiv Z \qquad \qquad \text{(Formula I)}$$

whereby W is a carbon atom or a sulfur atom and Z is an oxygen atom or a nitrogen atom, and (iv) the nucleic acid; b) optionally washing with a washing solution the substrate with the adsorbed nucleic acid, followed by c) contacting the substrate with the adsorbed nucleic acid with a solution containing salts in a lower concentration compared to the composition of step (a), thereby desorbing the nucleic acid from the substrate, and d) separating the solution with the desorbed nucleic acid from the substrate, thereby purifying the nucleic acid, and optionally (e) precipitating the desorbed nucleic acid from the solution of step (d) and isolating the precipitated nucleic acid, thereby further purifying the nucleic acid.

Also useful is a method for adsorbing a nucleic acid on a substrate, comprising the steps of (a) providing the nucleic acid in an aequous solution containing salts in a high concentration and a water-miscible non-acidic organic compound comprising a functional group of Formula I, whereby W is a carbon atom or a sulfur atom and Z is an oxygen atom or a nitrogen atom; followed by (b) adding the aequous solution of step (a) to the substrate.

Further useful is a method for adsorbing a nucleic acid on a substrate, comprising the steps of (a) providing the nucleic acid in an aequous solution containing salts in a high concentration; (b) providing the substrate in the form of powdered material; (c) providing a water-miscible non-acidic organic compound comprising a functional group of Formula I, whereby W is a carbon atom or a sulfur atom and Z is an oxygen atom or a nitrogen atom; followed by dispersing the substrate of step (b) in the water-miscible non-acidic organic compound of step (c) to form a suspension of said substrate; and (e) mixing the aequous solution of step (a) with the suspension of step (d).

Further useful is a suspension containing a substrate in the form of powdered material dispersed in a water-miscible non-acidic organic compound comprising a functional group of Formula I, whereby W is a carbon atom or a sulfur atom and Z is an oxygen atom or a nitrogen atom.

Further useful is the use of a water-miscible non-acidic organic compound comprising a functional group of Formula I, whereby W is a carbon atom or a sulfur atom and Z is an oxygen atom or a nitrogen atom, for performing the methods according to the invention described herein. Also useful is the use of a water-miscible non-acidic organic compound comprising a functional group of Formula I, whereby W is a carbon atom or a sulfur atom and Z is an oxygen atom or a nitrogen atom, for preparing a suspension by way of dispersing a substrate in said water-miscible non-acidic organic compound to form a suspension of said substrate. Further useful is the use of a suspension mentioned above for performing a method according to the invention.

Also useful are kits of parts containing a water-miscible non-acidic organic compound comprising a functional group of Formula I, whereby W is a carbon atom or a sulfur atom and Z is an oxygen atom or a nitrogen atom.

It has also been found that that nucleic acids can be bound to a substrate if the adsorption solution of high ionic strength contains a water-miscible cyclic diether.

Thus, further useful is a method for the purification of a nucleic acid, comprising the steps of: a) adsorbing on a substrate the nucleic acid from a composition containing (i) an aequous buffer, (ii) salts in a high concentration, (iii) a water-miscible cyclic diether, (iv) the nucleic acid; b) optionally washing with a washing solution the substrate with the adsorbed nucleic acid; followed by c) contacting the substrate with the adsorbed nucleic acid with a solution containing salts in a lower concentration compared to the composition of step (a), thereby desorbing the nucleic acid from the substrate; and (d) separating the solution with the desorbed nucleic acid from the substrate, thereby purifying the nucleic acid, and optionally (e) precipitating the desorbed nucleic acid from the solution of step (d) and isolating the precipitated nucleic acid, thereby further purifying the nucleic acid.

Further useful is a method for adsorbing a nucleic acid on a substrate, comprising the steps of (a) providing the nucleic acid in an aequous solution containing salts in a high concentration and a water-miscible cyclic diether; and (b) adding the aequous solution of step (a) to the substrate.

Also useful is a method for adsorbing a nucleic acid on a substrate, comprising the steps of (a) providing the nucleic acid in an aequous solution containing salts in a high concentration; (b) providing the substrate in the form of powdered material; (c) providing a water-miscible cyclic diether; (d) dispersing the substrate of step (b) in the water-miscible cyclic diether of step (c) to form a suspension of said substrate; and (e) mixing the aequous solution of step (a) with the suspension of step (d).

Further useful is a suspension containing a substrate in the form of powdered material dispersed in a water-miscible cyclic diether.

Also useful is the use of a water-miscible cyclic diether, for performing the methods according to the invention described herein. Further useful is the use of a water-miscible cyclic diether, for preparing a suspension by way of dispersing a substrate in said water-miscible non-acidic organic compound to form a suspension of said substrate. Also useful is the use of a suspension described herein for performing a method according to the invention.

Further useful are kits of parts containing a water-miscible cyclic diether.

Also useful is a method for determining the presence of a nucleic acid in a biological sample, comprising the steps of: (a) lysing the biological sample; (b) forming a composition containing (i) the lysed biological sample of step (a), (ii) an aqueous buffer, (iii) salts in a high concentration, (iv) a water-miscible non-acidic organic compound comprising a functional group of Formula I,

$$W \equiv Z \qquad \qquad \text{(Formula I)}$$

whereby W is a carbon atom or a sulfur atom and Z is an oxygen atom or a nitrogen atom; (c) contacting the composition of step (b) with a substrate, thereby adsorbing the nucleic acid to the substrate; (d) optionally washing with a washing solution the substrate with the adsorbed nucleic acid; followed by (e) contacting the substrate with the adsorbed nucleic acid with a solution containing salts in a lower concentration compared to the composition of step (a), thereby desorbing the nucleic acid from the substrate; and (f) separating the solution with the desorbed nucleic acid from the substrate; and (g) detecting in the solution of step (f) the presence of the nucleic acid, thereby determining the presence of the nucleic acid. Preferably, the nucleic acid is determined by amplification of the nucleic acid by means of the polymerase chain reaction using specific primers, a specific detection probe, and an amplification mixture, whereby amplification is monitored in real time.

Also useful is a method for determining the presence of a nucleic acid in a biological sample, comprising the steps of: (a) lysing the biological sample; (b) forming a composition containing (i) the lysed biological sample of step (a), (ii) an aqueous buffer, (iii) salts in a high concentration, (iv) a water-miscible cyclic diether, whereby W is a carbon atom or a sulfur atom and Z is an oxygen atom or a nitrogen atom; (c) contacting the composition of step (b) with a substrate, thereby adsorbing the nucleic acid to the substrate; (d) optionally washing with a washing solution the substrate with the adsorbed nucleic acid; followed by (e) contacting the substrate with the adsorbed nucleic acid with a solution containing salts in a lower concentration compared to the composition of step (a), thereby desorbing the nucleic acid from the substrate; and (f) separating the solution with the desorbed nucleic acid from the substrate; and (g) detecting in the solution of step (f) the presence of the nucleic acid, thereby determining the presence of the nucleic acid.

In the present document it is understood that the term "a nucleic acid" denotes at least one nucleic acid.

Furhermore, the term "a nucleic acid" also may indicate a mixture of nucleic acids. The term "nucleic acid" encompasses RNA, DNA, or both. The term "substrate" denotes a substance which is substantially insoluble in an aequous solution and on which a nucleic acid in an aequous solution of high ionic strength can adsorb when the substance is added. Examples therefore are porous or non-porous mineral particles such as silica, glass, quartz, zeolites or mixtures thereof. Also, the term "substrate" encompasses magnetically attractable particles coated with silica, glass, quartz, or zeolites. Further, it is understood that a substrate in the form of "powder" or "powdered" material refers to finely divided material which, when dispersed in a liquid phase such as a liquid organic compound or an aequous solution, produces a suspension. The term "powder" or "powdered" material is intended to include tablets, in which the powdered material has been aggregated, but still yields a suspension when combined with the liquid organic compound or the aequous solution.

Further, it is understood that the terms "high ionic strength" and "high concentration" mean the ionic strength or concentration in an aequous solution that results from dissolved salts in concentrations equal to or greater than about 1 M. Preferred are salts present in the aequous solution in concentrations of 1 to 10 M. More preferred are chaotropic salts in concentrations of 1 to 8 M. Further, the term "water-miscible" indicates that at room temperature and normal atmospheric pressure the non-aequous organic compound can be dissolved in water at a ratio equal or greater than 1% (per cent) volume by volume, to form a homogeneous liquid phase. The term "non-acidic" organic compound denotes an organic compound lacking a carboxy function.

In detail, the procedure for binding a (at least one) nucleic acid (also referred to as target nucleic acid) to a substrate such as, e.g., glass particles can be described as follows. It is preferably performed in the presence of chaotropic salts with a concentration of between 1 and 8 mol/l, and preferably between 2 and 6 mol/l. Chaotropic salts can be sodium iodide, sodium perchlorate, guanidine thiocyanate, guanidine isothiocyanate or guanidine hydrochloride. Other substances such as lithium chloride, sodium chloride, potassium chloride, sodium acetate, urea, and mixtures thereof are also possible.

The purification effect results from the behavior of DNA or RNA to bind to material with a glass surface under these conditions i.e. in the presence of certain concentrations of a chaotropic agent and, preferably, a water-miscible non-acidic organic compound. To bring the sample in contact with the substrate, i.e. the material with an affinity to nucleic acids, the sample is mixed with the material and incubated for a period of time sufficient for the binding to occur. Experts are usually familiar with the duration of the incubation step from procedures for performing treatment with non-magnetic particles. This step can be optimized by determining the quantity of immobilized biological material on the surface at different points in time. Incubation times of between 10 seconds and 30 minutes can be appropriate for nucleic acids. After incubation, the bound (at least one) target component, i.e. the nucleic acid(s) is separated from the liquid. This may be achieved in general by gravity or in the convenient case of nucleic acids bound to magnetic glass particles by separating the material bound to the magnetic particles by applying a magnetic field. For instance, the magnetic particles can be pulled to the wall of the vessel in which incubation was performed. The liquid containing the sample contents that were not bound to the magnetic particles can then be removed. The removal procedure used depends on the type of vessel in which incubation was performed. Suitable steps include removing the liquid via pipetting or aspiration.

Another example is binding the nucleic acid in the adsorption solution to a glass fleece. Commercial kits often provide such a fleece at the bottom of a column. The adsorption solution containing the nucleic acid is transferred to the column and passed through the fleece by applying force. The term "force" includes gravitational force and, preferred, centrifugal force. Very much preferred is the "spin column" procedure wherein the adsorption solution is passed through the filter due to force being applied by way of centrifugation. Other ways to pass the adsorption solution through the fleece include the application of pressure or suction.

The material with the bound DNA or RNA may then be washed at least once Preferably, the washing solution contains between more than 1 and less than 100 per cent volume by volume of the water-miscible non-acidic organic compound. Also preferred, the washing solution contains between 1 and 100 per cent volume by volume of the water-miscible non-acidic organic compound. More preferred, the washing solution is a mixture of 1-50% volume by volume of a water-miscible non-acidic organic compound in water. Another very much preferred washing solution is a mixture of 40-80% volume by volume of a water-miscible non-acidic organic compound with water. Another very much preferred washing solution is a mixture of 50-99% volume by volume of a water-miscible non-acidic organic compound with water. Even more preferred is a washing solution is a mixture of about 70% volume by volume of a water-miscible non-acidic organic compound with water. Also preferred for washing is the water-miscible non-acidic organic compound, that is to say the pure liquid compound as obtained from commercial suppliers is also understood as being encompassed by the term "washing solution".

A wash solution is used that does not cause the (at least one) target nucleic acid(s) to be released from the material surface but that washes away the undesired contaminants as thoroughly as possible. This wash step preferably takes place by incubating the material with the bound target nucleic acid(s) with the wash solution. The material is preferably resuspended during this step. Also preferred, in case the material is a glass fleece or a packing in a column, the washing step takes place by rinsing the column with the washing solution. Preferably, the washing solution is passed through the column by applying pressure, suction, centrifugal force or gravitational force. The above equally applies when the water-miscible non-acidic organic compound is used in pure form.

The contaminated wash solution is preferably removed just as in the step described above for binding the nucleic acid to the substrate material. After the last washing step, the material can be dried briefly in a vacuum, or the fluid can be allowed to evaporate. A pretreatment step using acetone may also be performed.

Afterwards, the conditions may be reversed, e.g. the concentration of the chaotropic agent or the water-miscible non-acidic organic compound is decreased to elute the DNA or RNA bound to the material. Preferably, the process of separating the substrate, e.g. the magnetic glass particles, from the rest of the sample is done by pelleting the immobilized biological material, e.g. by gravity force or by the use of a magnet in the case of magnetic glass particles and removal of the supernatant. Then the magnetic glass particles with the immobilized biological material are resuspended in an aequous solution with no or only a low amount of chaotropic agent and/ or water-miscible non-acidic organic compound. Alternatively, the suspension can be diluted with a solution with no or only a low amount of chaotropic agent and/ or water-miscible non-acidic organic compound. Buffers of this nature are known from DE 37 24 442 and Jakobi, R., et al., Anal. Biochem. 175 (1988) 196-201. The elution buffers with a low salt content are in particular buffers with a content of less than 0.2 mol/l. Preferably, the elution buffer contains the substance Tris for buffering purposes. Also preferred, the elution buffer is demineralized water. The solution containing purified DNA or RNA can now be used for other reactions. Optionally, the nucleic acid(s) can be precipitated from the solution using, e.g., ethanol or isopropanol. The precipitate can also be subjected to further washing steps. Methods of this kind are well known to the skilled artisan and are described in detail in Sambrook, Fritsch & Maniatis, Molecular Cloning, A Laboratory Manual, 3rd edition, CSHL Press, 2001.

For the washing steps in the methods of the invention, preferably liquids are used which are suitable for processes in molecular biology, in particular desoxyribonucleic acid (DNA) or ribonucleic acid (RNA) purification processes which make use of the binding of these substances to glass particles under certain conditions. Preferred liquids comprise a water-miscible non-acidic organic compound comprising a functional group of Formula I,

$$W \equiv Z \qquad \text{(Formula I)}$$

whereby W is a carbon atom or a sulfur atom and Z is an oxygen atom or a nitrogen atom. Preferably, the functional group is selected from the group consisting of an oxo group, a sulfoxo group, a cyano group, and a carbonyl group of a carbamoyl function or an amide but not belonging to a carboxy function. More preferred, the water-miscible non-acidic organic compound is selected from the group consisting of acetone, acetylacetone, acetonitrile, dimethylsulfoxide, diethylketone, methylethylketone, methylpropylketone, isobutylmethylketone, gamma-butyrolactone, gamma-valerolactone, propylene carbonate, and N-methyl-2-pyrrolidone. Also useful are liquids comprising as a water-miscible non-acidic organic compound a cyclic diether. Preferably, the cyclic diether is dioxane.

The magnetic glass particles used in the present invention may be provided in different formulations. It is possible to provide them in the form of a tablet, as a powder or as a suspension. Very much preferred, the magnetic glass particles are suspended in the water-miscible non-acidic organic compound. Preferably, these suspensions contain between 5 to 60 mg/ml magnetic glass particles (MGPs). Also preferred, the silica-containing material is suspended in aqueous buffered solutions which may optionally contain a chaotropic agent in a concentration of between 2 and 8 mol/l, and preferably between 4 and 6 mol/l. Chaotropic salts are sodium iodide, sodium perchlorate, guanidine thiocyanate, guanidine isothiocyanate or guanidine hydrochloride. Other compounds known to the skilled artisan are also possible. A chaotropic agent according to the present invention is any chemical substance which disturbs the ordered structure of liquid water and has the effect that DNA or RNA binds to the magnetic glass particles if this agent is present in the DNA or RNA containing solution. It is obvious for the artisan to produce suitable aqueous buffered solutions. Buffer systems which suitable for molecular biology purposes may be found e.g. in Sambrook, Fritsch & Maniatis, Molecular Cloning, A Laboratory Manual, 3rd edition, CSHL Press, 2001. Preferred buffer substances are Tris-(hydroxymethyl)-aminomethane (TRIS), phosphate, N-(2-Hydroxyethyl)piperazine-N'-(2-ethanesulfonic acid) (HEPES), salts thereof or other suitable substances. Additionally, substances may be present which modify the ionic strength of the solution as e.g. NaCl, KCl or CaCl$_2$ or which are metal cation complexing agents as e.g. ethylene-diamine-tetra-acetic acid (EDTA) or the salts thereof. Other biological substances known to the skilled artisan may also be present.

The method according to the present invention is suitable for the purification of nucleic acids, i.e. RNA or DNA, from complex mixtures with other biological substances containing them. Thereby also mixtures of different nucleic acids may be purified, even mixtures containing a nucleic acid of interest in low abundance. Thus, the present invention also encompasses the purification of mixtures of specific nucleic acids in which the target nucleic acid(s) may be a minor component in terms of concentration (or may be present in low abundance).

The procedure described can be used to isolate native or modified nucleic acids. Native nucleic acids are understood to be nucleic acids, the structure of which was not irreversibly changed compared with the naturally-occurring nucleic acids. This does not mean that other components of the sample can not be modified, however. Modified nucleic acids include nucleic acids that do not occur in nature, e.g., nucleic acids that are modified by attaching to them groups that are reactive, detectable or capable of immobilization. An example of this are biotinylated nucleic acids.

After the steps described above, the nucleic acids isolated using the methods according to the invention can now be used further as necessary. For instance, they can be used as a substrate for various enzymatic reactions. When nucleic acids are involved, they can be used for sequencing, radioactive or non-radioactive labelling, amplification of one or more of the sequences they contain, transcription, hybridization with labelled probe nucleic acids, translation or ligation. Therefore, the invention also encompasses the method comprising the step of releasing the bound target nucleic acids from the material with an affinity thereto. If desired, the target nucleic acid(s) purified in this manner can be separated from the material as described above.

Therefore, an embodiment of the invention is a method for the purification of a nucleic acid, comprising the steps of:

(a) adsorbing on a porous or non-porous mineral substrate selected from the group consisting of silica gel, glass fibers, quartz fibers, zeolites, and magnetically attractable particles coated with glass the nucleic acid $W \equiv Z$ sequence-independently from a composition containing

(i) an aqueous buffer,

(ii) salts in a concentration equal to or greater than 1 M,

(iii) a water-miscible non-acidic organic compound comprising a functional group of Formula I,

$$W \equiv Z \qquad \qquad (Formula\ I)$$

whereby W is a carbon atom or a sulfur atom and Z is an oxygen atom or a nitrogen atom,
whereby the functional group is selected from the group consisting of an oxo group, a sulfoxo group, a cyano group, and a carbonyl group of a carbamoyl function or an amide but not belonging to a carboxy function, and the water-miscible non-acidic organic compound is selected from the group consisting of acetylacetone, dimethylsulfoxide, diethylketone, methylethylketone, methylpropylketone, isobutylmethylketone, gamma-butyrolactone, gamma-valerolactone, propylene carbonate, and N-methyl-2-pyrrolidone, and
whereby composition of step (a) contains 1 to 50 per cent volume by volume of the water-miscible non-acidic organic compound, and the water-miscible non-acidic organic compound is dissolved to form a homogeneous liquid phase,

(iv) the nucleic acid;

(b) optionally washing with a washing solution the substrate with the adsorbed nucleic acid; followed by

(c) contacting the substrate with the adsorbed nucleic acid with a solution containing salts in a lower concentration compared to the composition of step (a), thereby desorbing the nucleic acid from the substrate; and

(d) separating the solution with the desorbed nucleic acid from the substrate, thereby purifying the nucleic acid; and optionally

(e) precipitating the desorbed nucleic acid from the solution of step (d) and isolating the precipitated nucleic acid, thereby further purifying the nucleic acid.

It is also contemplated that the composition of step (a) is made use of in automated processes for the purification of a (at least one) nucleic acid. Automatic processing devices capable of performing the methods of the invention, such as robots with a pipetting device, often have open vessels that contain solutions, e.g. stock solutions. In this regard it is advantageous that the liquid phases of the solutions contain a solvent which under normal atmospheric pressure and room temperature has a low tendency to evaporate. Therefore, very much preferred, the water-miscible non-acidic organic compound is dimethylsulfoxide. Also very much preferred, the water-miscible non-acidic organic compound is N-methyl-2-pyrrolidone.

Preferably, the composition of step (a) contains 2 to 35 per cent volume by volume of the water-miscible non-acidic organic compound. Even more preferred, the composition of step (a) contains 3 to 30 per cent volume by volume of the water-miscible non-acidic organic compound. Very much preferred, the composition of step (a) contains about 4 per cent volume by volume of the water-miscible non-acidic organic compound. Very much preferred, the composition of step (a) contains about 15 per cent volume by volume of the water-miscible non-acidic organic compound. Very much preferred, the composition of step (a) contains about 25 per cent volume by volume of the water-miscible non-acidic organic compound.

Preferably, the salts in the composition of step (a) are chaotropic salts in concentrations of 1 to 8 M. More preferred, said chaotropic salts are selected from the group consisting of sodium perchlorate, guanidine hydrochloride, guanidine thiocyanate, guanidine isothiocyanate, and sodium iodide.

Also preferred, the salts in the composition of step (a) are in concentrations of 1 to 10 M and said salts are selected from the group consisting of lithium chloride, sodium chloride, potassium chloride, sodium acetate, urea, and mixtures thereof.

Preferably, the washing solution contains the water-miscible non-acidic organic compound. More preferred, the washing solution contains 1 to 50 per cent volume by volume of the water-miscible non-acidic organic compound. Even more preferred, the washing solution contains 2 to 35 per cent volume by volume of the water-miscible non-acidic organic compound. Even more preferred, the washing solution contains 3 to 30 per cent volume by volume of the water-miscible non-acidic organic compound. Very much preferred, the washing solution contains about 4 per cent volume by volume of the water-miscible non-acidic organic compound. Very much preferred, the washing solution contains about 15 per cent volume by volume of the water-miscible non-acidic organic compound. Very much preferred, the washing solution contains about 25 per cent volume by volume of the water-miscible non-acidic organic compound.

Preferably, the substrate comprises a porous or non-porous mineral substrate selected from the group consisting of silica gel, glass fibers, quartz fibers, and zeolites. Also preferred, the substrate comprises a porous or non-porous mineral substrate selected from the group consisting of metal oxides, and/ or metal mixed oxides, alumina, titania, zirconia, and materials predominantly consisting of glass. It is also preferred that the mineral substrate has a particle size of 0.1 $\mu$m to 1,000 $\mu$m. It is also preferred that porous mineral support materials, when employed, have a pore size of from 2 to 1,000 nm. More preferred, porous or non-porous support materials, especially zeolites, are in the form of loose packings. Even more preferred, the mineral substrate consists of filter sheets in the form of glass, quartz or ceramic filter sheets, and/ or a membrane containing silica gel and/ or particles or fibers of mineral supports and fabrics of quartz or glass wool. It is also preferred that the substrate comprises magnetically attractable particles. More preferred, the magnetically attractable particles are coated with a mineral substrate selected from the group consisting of silica gel, glass, quartz, and zeolites. Even more preferred, the substrate comprises magnetically attractable particles coated with glass. The target nucleic acid(s) can be detected and determined. The above-described purification method is preferred, followed by a determination or detection step or purification methods followed by an amplification and determination or detection step. The target nucleic acid or nucleic acids of interest may be contained in a matrix of non-target nucleic acids, and may even be a minor component in said mixture of specific nucleic acids. Suitable DNA detection methods are known to the skilled artisan and are described in standard textbooks as Sambrook, Fritsch & Maniatis, Molecular Cloning, A Laboratory Manual, 3rd edition, CSHL Press, 2001; and Ausubel et al., Current Protocols in Molecular Biology, J. Wiley and Sons, NY, 1987. There may be also further purification steps before the DNA detection step is carried out as e.g. a precipitation step. The detection methods may include but are not limited to the binding or intercalating of specific dyes as ethidiumbromide which intercalates into the double-stranded DNA and changes its fluorescence thereafter. The purified DNA may also be separated by electrophoretic methods, optionally after a restriction digest, and visualized thereafter. There are also probe-based assays which exploit the oligonucleotide hybridization to specific sequences and subsequent detection of the hybrid. It is also possible to sequence the DNA after further steps known to the skilled artisan. Other methods apply a diversity of DNA sequences to a silicon chip to which specific probes are bound and yield a signal when a complementary sequences bind.

The invention also encompasses the mixture of non-proteinaceous and proteinaceous components comprising nucleic acids whereby the nucleic acids comprise DNA or RNA or both.

The invention also encompasses biological samples, from which nucleic acids are purified, comprising viruses or bacterial cells, as well as isolated cells from multicellular organisms as e.g. human and animal cells such as leucocytes, and immunologically active low and high molecular chemical compounds such as haptens, antigens, antibodies and nucleic acids, blood plasma, cerebral fluid, sputum, stool, biopsy specimens, bone marrow, oral rinses, blood serum, tissues, urine or mixtures thereof. The present invention also encompasses biological samples such as a fluid from the human or animal body; preferably the biological sample is blood, blood plasma, blood serum or urine. The blood plasma is preferably EDTA, heparin or citrate blood plasma. In an embodiment of the invention the biological sample comprises bacterial cells, eukaryotic cells, viruses or mixtures thereof. A biological sample as exemplified above, preferably in a processed form such as a lysate, can be part of the composition from which the (target) nucleic acid is adsorbed to the substrate.

It is also preferred that the mixture of nucleic acids and proteinaceous material comprises desoxyribonucleic acid (DNA) or ribonucleic acid (RNA) or both, preferably the DNA or RNA or both is derived from a virus or a (at least one) microorganism. The virus can be hepatitis A virus (HAV), hepatitis B virus (HBV), hepatitis C virus (HCV), the human immunodeficiency virus (HIV), the human papilloma virus (HPV) or parvovirus B19.

It is also preferred that a target nucleic acid component and the other nucleic acids are purified essentially as described above. Then the target nucleic acid component is further manipulated and detected, i.e. it is amplified with the polymerase chain reaction which specifically amplifies target sequences to detectable amounts. Other possible amplification reactions are the ligase Chain Reaction (LCR, Wu, D.Y., and Wallace, R.B., Genomics 4 (1989) 560-569, and Barany, F., Proc. Natl. Acad. Sci. USA 88 (1991) 189-193); Polymerase Ligase Chain Reaction (Barany, F., PCR Methods and Applic. 1 (1991) 5-16); Gap-LCR (PCT Patent Publication No. WO90/01069); Repair Chain Reaction (European Patent Publication No. EP 0 439 182 A2), 3SR (Kwoh, D.Y., et al., Proc. Natl. Acad. Sci. USA 86 (1989) 1173-1177; Guatelli, J.C., et al., Proc. Natl. Acad. Sci. USA 87 (1990) 1874-1878; PCT Patent Publication No. WO 92/08800), and NASBA (U.S. Pat. No. 5,130,238). Further, there are strand displacement amplification (SDA), transciption mediated amplification (TMA), and Q-beta-amplification (for a review see e.g. Whelen, A.C., and Persing, D.H., Annu. Rev. Microbiol. 50 (1996) 349-373; Abramson, R.D., and Myers, T.W., Curr. Opin. Biotechnol. 4 (1993) 41-47).

Particularly preferred is the TaqMan® detection method disclosed in WO 92/02638 and the corresponding US patents US 5,210,015; US 5,804,375; US 5,487,972. This method exploits the exonuclease activity of a polymerase to generate a signal. In detail, the target nucleic acid component is detected by a process comprising contacting the sample with an oligonucleotide containing a sequence complementary to a region of the target nucleic acid component and a labelled oligonucleotide containing a sequence complementary to a second region of the same target nucleic acid component sequence strand, but not including the nucleic acid sequence defined by the first oligonucleotide, to create a mixture of duplexes during hybridization conditions, wherein the duplexes comprise the target nucleic acid annealed to the first oligonucleotide and to the labelled oligonucleotide such that the 3'-end of the first oligonucleotide is adjacent to the 5'-end of the labelled oligonucleotide. Then this mixture is treated with a template-dependent nucleic acid polymerase having a 5' to 3' nuclease activity under conditions sufficient to permit the 5' to 3' nuclease activity of the polymerase to cleave the annealed, labelled oligonucleotide and release labelled fragments. The signal generated by the hydrolysis of the labelled oligonucleotide is detected and/ or measured. TaqMan® technology eliminates the need for a solid phase bound reaction complex to be formed and made detectable. In more general terms, a procedure for the purification of a target nucleic acid component followed by a detection step is disclosed wherein the amplification and/ or detection reaction is a homogeneous solution-phase.

Useful is a suspension containing a substrate in the form of powdered material dispersed in a water-miscible non-acidic organic compound comprising a functional group of Formula I,

$$W \equiv Z \qquad \text{(Formula I)}$$

whereby W is a carbon atom or a sulfur atom and Z is an oxygen atom or a nitrogen atom. Preferably, the functional group is selected from the group consisting of an oxo group, a sulfoxo group, a cyano group, and a carbonyl group of a carbamoyl function or an amide but not belonging to a carboxy function. More preferred, the water-miscible non-acidic organic compound is selected from the group consisting of acetone, acetylacetone, acetonitrile, dimethylsulfoxide, diethylketone, methylethylketone, methylpropylketone, isobutylmethylketone, gamma-butyrolactone, gamma-valerolactone, propylene carbonate, and N-methyl-2-pyrrolidone. It is also useful that the suspension described above is used in automated processes for the purification of a (at least one) nucleic acid. Automatic processing devices capable of performing a method of the invention, such as robots with a pipetting device, often have open vessels that contain solutions, e.g. stock solutions, and suspensions such as the suspension mentioned above. In this regard it is advantageous that the liquid phases of the solutions and/ or suspensions contain a solvent which under normal atmospheric pressure and room temperature has a low tendency to evaporate. Therefore, very much preferred, the water-miscible non-acidic organic compound is dimethylsulfoxide. Also very much preferred, the water-miscible non-acidic organic compound is N-methyl-2-pyrrolidone.

Preferably, the substrate comprises a powdered porous or non-porous mineral substrate selected from the group consisting of silica gel, glass, quartz, and zeolites. Also preferred, the substrate comprises a powdered porous or non-porous mineral substrate selected from the group consisting of metal oxides, and/ or metal mixed oxides, alumina, titania, zirconia, and materials predominantly consisting of glass. It is also preferred that the substrate comprises magnetically attractable particles. More preferred, the magnetically attractable particles are coated with a mineral substrate selected from the group consisting of silica gel, glass, quartz, and zeolites. Even more preferred, the substrate comprises magnetically attractable particles coated with glass.

Also useful is the use of a water-miscible non-acidic organic compound comprising a functional group of Formula I,

$$W \equiv Z \qquad \text{(Formula I)}$$

whereby W is a carbon atom or a sulfur atom and Z is an oxygen atom or a nitrogen atom, for performing the methods according to the invention described herein.

Preferably, the functional group is selected from the group consisting of an oxo group, a sulfoxo group, a cyano group, and a carbonyl group of a carbamoyl function or an amide but not belonging to a carboxy function. More preferred, the water-miscible non-acidic organic compound is selected from the group consisting of acetone, acetylacetone, acetonitrile, dimethylsulfoxide, diethylketone, methylethylketone, methylpropylketone, isobutylmethylketone, gamma-butyrolactone, gamma-valerolactone, propylene carbonate, and N-methyl-2-pyrrolidone. It is also useful that the suspension described above is used in automated processes for the purification of a (at least one) nucleic acid. Automatic processing devices capable of performing a method of the invention, such as robots with a pipetting device, often have open vessels that contain solutions, e.g. stock solutions, and suspensions such as the suspension mentioned above. In this regard it is advantageous that the liquid phases of the solutions and/ or suspensions contain a solvent which under normal atmospheric pressure and room temperature has a low tendency to evaporate. Therefore, very much preferred, the water-miscible non-acidic organic compound is dimethylsulfoxide. Also very much preferred, the water-miscible non-acidic organic compound is N-methyl-2-pyrrolidone.

Further useful is the use of a water-miscible non-acidic organic compound comprising a functional group of Formula I,

$$W \equiv Z \qquad \text{(Formula I)}$$

whereby W is a carbon atom or a sulfur atom and Z is an oxygen atom or a nitrogen atom, for preparing a suspension by way of dispersing a substrate in said water-miscible non-acidic organic compound to form a suspension of said substrate.

Preferably, the functional group is selected from the group consisting of an oxo group, a sulfoxo group, a cyano group, and a carbonyl group of a carbamoyl function or an amide but not belonging to a carboxy function. More preferred, the water-miscible non-acidic organic compound is selected from the group consisting of acetone, acetylacetone, acetonitrile, dimethylsulfoxide, diethylketone, methylethylketone, methylpropylketone, isobutylmethylketone, gamma-butyrolactone, gamma-valerolactone, propylene carbonate, and N-methyl-2-pyrrolidone. It is also useful that the suspension described above is used in automated processes for the purification of a (at least one) nucleic acid. Automatic processing devices capable of performing a method of the invention, such as robots with a pipetting device, often have open vessels that contain solutions, e.g. stock solutions, and suspensions such as the suspension mentioned above. In this regard it is advantageous that the liquid phases of the solutions and/ or suspensions contain a solvent which under normal atmospheric pressure and room temperature has a low tendency to evaporate. Therefore, very much preferred, the water-miscible non-acidic organic compound is dimethylsulfoxide. Also very much preferred, the water-miscible non-acidic organic compound is N-methyl-2-pyrrolidone.

Preferably, the substrate comprises a powdered porous or non-porous mineral substrate selected from the group consisting of silica gel, glass, quartz, and zeolites. Also preferred, the substrate comprises a powdered porous or non-porous mineral substrate selected from the group consisting of metal oxides, and/ or metal mixed oxides, alumina, titania, zirconia, and materials predominantly consisting of glass. It is also preferred that the substrate comprises magnetically attractable particles. More preferred, the magnetically attractable particles are coated with a mineral substrate selected from the group consisting of silica gel, glass, quartz, and zeolites. Even more preferred, the substrate comprises magnetically attractable particles coated with glass.

Further useful is the use of a suspension described above for performing a method according to the invention as described herein.

Also useful are kits. Such kits known in the art further comprise plastics ware which can be used during the sample preparation procedure as e.g. microtitre plates in the 96 or 384 well format or just ordinary reaction tubes manufactured e.g. by Eppendorf, Hamburg, Germany, and all other reagents for carrying out the methods according to the invention. Therefore, the kit can additionally contain a material with an affinity to nucleic acids (and the (at least one) target nucleic acid component), preferably the material with an affinity to nucleic acids (and the (at least one) target nucleic acid component) comprises a material with a silica surface. Preferably, the material with a silica surface is a glass. Most preferably, the material with an affinity to nucleic acids is a composition comprising magnetic glass particles, i.e. magnetically attractable particles coated with glass. Another preferred material with an affinity to nucleic acids is an anion exchanger. The kit can further or additionally comprise a lysis buffer containing e.g. chaotropic agents, detergents or mixtures thereof which allows the lysis of cells. These components of the kit described above may be provided separately in tubes or storage containers. Depending on the nature of the components, these may be even provided in a single tube or storage container. The kit may further or additionally comprise a washing solution which is suitable for the washing step of the magnetic glass particles when DNA or RNA is bound thereto. This washing solution may contain a water-miscible non-acidic organic compound described herein and/ or chaotropic agents in a buffered solution or solutions with an acidic pH without a water-miscible non-acidic organic compound described herein and/ or chaotropic agents as described above. Often the washing solution or other solutions are provided as stock solutions which have to be diluted before use. The kit may further or additionally comprise an eluent or elution buffer, i.e. a solution or a buffer (e.g. 10 mM Tris, 1 mM EDTA, pH 8.0) or pure water to elute the DNA or RNA bound to the magnetic glass particles. Further, additional reagents or buffered solutions may be present which can be used for the purification process of a nucleic acid, i.e. DNA or RNA.

Also useful is a kit of parts, containing (a) a concentrated stock solution of a buffer salt and a chaotropic salt selected from the group consisting of sodium perchlorate, guanidine hydrochloride, guanidine thiocyanate, guanidine isothiocyanate, and sodium iodide; (b) a water-miscible non-acidic organic compound comprising a functional group of Formula I,

$$W \equiv Z \qquad \text{(Formula I)}$$

whereby W is a carbon atom or a sulfur atom and Z is an oxygen atom or a nitrogen atom and the water-miscible non-acidic organic compound is selected from the group consisting of acetone, acetylacetone, acetonitrile, dimethylsulfoxide, diethylketone, methylethylketone, methylpropylketone, isobutylmethylketone, gamma-butyrolactone, gamma-valerolactone, propylene carbonate, and N-methyl-2-pyrrolidone; (c) buffer solutions; and (d) chromatographic and filtering material.

Further useful is a kit of parts, containing (a) a concentrated stock solution of a buffer salt and a salt selected from the group consisting of lithium chloride, sodium chloride, potassium chloride, sodium acetate, urea, and mixtures thereof; (b) a water-miscible non-acidic organic compound comprising a functional group of Formula I,

$$W \equiv Z \qquad \text{(Formula I)}$$

whereby W is a carbon atom or a sulfur atom and Z is an oxygen atom or a nitrogen atom and the water-miscible non-acidic organic compound is selected from the group consisting of acetone, acetylacetone, acetonitrile, dimethylsulfoxide, diethylketone, methylethylketone, methylpropylketone, isobutylmethylketone, gamma-butyrolactone, gamma-valerolactone, propylene carbonate, and N-methyl-2-pyrrolidone; (c) buffer solutions; and (d) chromatographic and filtering material.

Further useful is a kit of parts, containing (a) a concentrated stock solution of a buffer salt and a chaotropic salt selected from the group consisting of sodium perchlorate, guanidine hydrochloride, guanidine thiocyanate, guanidine isothiocyanate, and sodium iodide; (b) a suspension described above; (c) buffer solutions.

Further useful is a kit of parts, containing (a) a concentrated stock solution of a buffer salt and a salt selected from the group consisting of lithium chloride, sodium chloride, potassium chloride, sodium acetate, urea, and mixtures thereof; (b) a suspension described above; (c) buffer solutions.

A preferred embodiment of the present invention is to use the methods of the present invention in automatable methods as e.g. described in WO 99/16781. Automatable method means that the steps of the method are suitable to be carried out with an apparatus or machine capable of operating with little or no external control or influence by a human being. Automatized method means that the steps of the automatable method are carried out with an apparatus or machine capable of operating with little or no external control or influence by a human being. Only the preparation steps for the method may have to be done by hand, e.g. the storage containers have to filled up and put into place, the choice of the samples has to be done by a human being and further steps known to the expert in the field, e.g. the operation of the controlling computer. The apparatus or machine may e.g. add automatically liquids, mix the samples or carry out incubation steps at specific temperatures. Typically, such a machine or apparatus is a robot controlled by a computer which carries out a program in which the single steps and commands are specified. Preferred automatized methods are those which are carried out in a high-throughput format which means that the methods and the used machine or apparatus are optimized for a high-throughput of samples in a short time. In another embodiment of the invention the methods according to the present invention are used in semi-automatized process which means that some reaction steps may have to be done manually. In a preferred embodiment of the invention, a suspension containing MGPs (magnetic glass particles) is taken from a storage container and partial volumes are added to different reaction vessels. Reaction vessels may be reaction tubes made from plastics eventually in mictrotitreplate format contain 96 or 384 or more wells where a reaction can be carried out. However, these vessels may be made from other material, e.g. from steel.

It is clear to the skilled artisan that some of the organic compounds contemplated by the invention are capable of dissolving certain plastic materials. Thus, when determining the nature of suitable storage or reaction vessels, the skilled artisan will determine in a limited number of obvious experiments the material which is suited best for executing the methods of the invention or for producing kits.

The kits described herein are used for the purification of nucleic acids in research, bioanalytics or diagnostics. In preferred embodiments according to the invention the methods according to the invention are used in a high-throughput format, i.e. in an automatized method which allows the analysis of a high number of different samples in a very short time.

Further useful is a method for determining the presence of a nucleic acid in a biological sample, comprising the steps of: (a) lysing the biological sample; (b) forming a composition containing (i) the lysed biological sample of step (a), (ii) an aqueous buffer, (iii) salts in a high concentration, (iv) a water-miscible non-acidic organic compound comprising a functional group of Formula I,

$$W \equiv Z \qquad \text{(Formula I)}$$

whereby W is a carbon atom or a sulfur atom and Z is an oxygen atom or a nitrogen atom; (c) contacting the composition of step (b) with a substrate, thereby adsorbing the nucleic acid to the substrate; (d) optionally washing with a washing solution the substrate with the adsorbed nucleic acid; followed by (e) contacting the substrate with the adsorbed nucleic acid with a solution containing salts in a lower concentration compared to the composition of step (a), thereby desorbing the nucleic acid from the substrate; and (f) separating the solution with the desorbed nucleic acid from the substrate; and (g) detecting in the solution of step (f) the presence of the nucleic acid, thereby determining the presence of the nucleic acid. Preferably, the nucleic acid is determined by amplification of the nucleic acid by means of the polymerase chain reaction using specific primers, a specific detection probe, and an amplification mixture, whereby amplification is monitored in real time. Also preferred is to determine the nucleic acid by hybridizing the nucleic acid to a hybridization probe and detecting and/or quantifying the hybrid. The skilled artisan is aware of the fact that not only a nucleic acid can serve as a hybridization probe but also a nucleic acid comprising one or more nucleoside analogues can be used. In addition, nucleic acid analogues such as PNA are known to the art as being capable of forming detectable hybrids with nucleic acids. It is understood that the nucleic acid to be determined is DNA or RNA. Very much preferred is the above method, whereby the nucleic acid is RNA and step (g) comprises (i) reverse transcribing the RNA to form a cDNA, (ii) subsequently amplifying, by means of the polymerase chain reaction, the cDNA, (iii) detecting the presence of the cDNA, thereby determining the presence of the nucleic acid.

It has also been found by the inventors that a water-miscible cyclic diether is a suitable non-acidic organic compound to perform the methods according to the invention.

Therefore, also useful is a method for the purification of a nucleic acid, comprising the steps of: a) adsorbing on a substrate the nucleic acid from a composition containing (i) an aequous buffer, (ii) salts in a high concentration, (iii) a water-miscible cyclic diether, and (iv) the nucleic acid; b) optionally washing with a washing solution the substrate with the adsorbed nucleic acid; followed by c) contacting the substrate with the adsorbed nucleic acid with a solution containing salts in a lower concentration compared to the composition of step (a), thereby desorbing the nucleic acid from the substrate; and d) separating the solution with the desorbed nucleic acid from the substrate, thereby purifying the nucleic acid; and optionally (e) precipitating the desorbed nucleic acid from the solution of step (d) and isolating the precipitated nucleic acid, thereby further purifying the nucleic acid. Preferably, the water-miscible cyclic diether is dioxane.

Preferably, the composition of step (a) contains 1 to 50 per cent volume by volume of the water-miscible cyclic diether. More preferred, the composition of step (a) contains 2 to 35 per cent volume by volume of the water-miscible cyclic diether. Even more preferred, the composition of step (a) contains 3 to 30 per cent volume by volume of the water-miscible cyclic diether. Very much preferred, the composition of step (a) contains about 4 per cent volume by volume of the water-miscible cyclic diether. Very much preferred, the composition of step (a) contains about 15 per cent volume by volume of the water-miscible cyclic diether. Very much preferred, the composition of step (a) contains about 25 per cent volume by volume of the water-miscible cyclic diether.

Preferably, the salts in the composition of step (a) are chaotropic salts in concentrations of 1 to 8 M. More preferred, said chaotropic salts are selected from the group consisting of sodium perchlorate, guanidine hydrochloride, guanidine thiocyanate, guanidine isothiocyanate, and sodium iodide.

Also preferred, the salts in the composition of step (a) are in concentrations of 1 to 10 M and said salts are selected from the group consisting of lithium chloride, sodium chloride, potassium chloride, sodium acetate, urea, and mixtures thereof.

Preferably, the washing solution contains the water-miscible cyclic diether. More preferred, the washing solution contains 1 to 50 per cent volume by volume of the water-miscible cyclic diether. Even more preferred, the washing solution contains 2 to 35 per cent volume by volume of the water-miscible cyclic diether. Even more preferred, the washing solution contains 3 to 30 per cent volume by volume of the water-miscible cyclic diether. Very much preferred, the washing solution contains about 4 per cent volume by volume of the water-miscible cyclic diether. Very much preferred, the washing solution contains about 15 per cent volume by volume of the water-miscible cyclic diether. Very much preferred, the washing solution contains about 25 per cent volume by volume of the water-miscible cyclic diether.

Preferably, the substrate comprises a porous or non-porous mineral substrate selected from the group consisting of silica gel, glass fibers, quartz fibers, and zeolites. Also preferred, the substrate comprises a porous or non-porous mineral substrate selected from the group consisting of metal oxides, and/ or metal mixed oxides, alumina, titania, zirconia, and materials predominantly consisting of glass. It is also preferred that the mineral substrate has a particle size of 0.1 $\mu$m to 1,000 $\mu$m. It is also preferred that porous mineral support materials, when employed, have a pore size of from 2 to 1,000 nm. More preferred, porous or non-porous support materials, especially zeolites, are in the form of loose packings. Even more preferred, the mineral substrate consists of filter sheets in the form of glass, quartz or ceramic filter sheets, and/ or a membrane containing silica gel and/ or particles or fibers of mineral supports and fabrics of quartz or glass wool. It is also preferred that the substrate comprises magnetically attractable particles. More preferred, the magnetically attractable particles are coated with a mineral substrate selected from the group consisting of silica gel, glass, quartz, and zeolites. Even more preferred, the substrate comprises magnetically attractable particles coated with glass.

Further useful is a method for adsorbing a nucleic acid on a substrate, comprising the steps of (a) providing the nucleic acid in an aequous solution containing salts in a high concentration and a water-miscible cyclic diether; and (b) adding the aequous solution of step (a) to the substrate. Preferably, the water-miscible cyclic diether is dioxane.

Preferably, the aequous solution of step (a) contains 1 to 50 per cent volume by volume of the water-miscible cyclic diether. More preferred, the aequous solution of step (a) contains 2 to 35 per cent volume by volume of the water-miscible cyclic diether. Even more preferred, the aequous solution of step (a) contains 3 to 30 per cent volume by volume of the water-miscible cyclic diether. Very much preferred, the aequous solution of step (a) contains about 4 per cent volume by volume of the water-miscible cyclic diether. Very much preferred, the aequous solution of step (a) contains about 15 per cent volume by volume of the water-miscible cyclic diether. Very much preferred, the aequous solution of step (a) contains about 25 per cent volume by volume of the water-miscible cyclic diether.

Preferably, the salts in the aequous solution of step (a) are chaotropic salts in concentrations of 1 to 8 M. More preferred, said chaotropic salts are selected from the group consisting of sodium perchlorate, guanidine hydrochloride, guanidine thiocyanate, guanidine isothiocyanate, and sodium iodide.

Also preferred, the salts in the aequous solution of step (a) are in concentrations of 1 to 10 M and said salts are selected from the group consisting of lithium chloride, sodium chloride, potassium chloride, sodium acetate, urea, and mixtures thereof.

Preferably, the substrate comprises a porous or non-porous mineral substrate selected from the group consisting of silica gel, glass fibers, quartz fibers, and zeolites. Also preferred, the substrate comprises a porous or non-porous mineral substrate selected from the group consisting of metal oxides, and/ or metal mixed oxides, alumina, titania, zirconia, and materials predominantly consisting of glass. It is also preferred that the mineral substrate has a particle size of 0.1 $\mu$m to 1,000 $\mu$m. It is also preferred that porous mineral support materials, when employed, have a pore size of from 2 to 1,000 nm. More preferred, porous or non-porous support materials, especially zeolites, are in the form of loose packings. Even more preferred, the mineral substrate consists of filter sheets in the form of glass, quartz or ceramic filter sheets, and/ or a membrane containing silica gel and/ or particles or fibers of mineral supports and fabrics of quartz or glass wool. It is also preferred that the substrate comprises magnetically attractable particles. More preferred, the magnetically attractable particles are coated with a mineral substrate selected from the group consisting of silica gel, glass, quartz, and zeolites. Even more preferred, the substrate comprises magnetically attractable particles coated with glass.

Further useful is a method for adsorbing a nucleic acid on a substrate, comprising the steps of (a) providing the nucleic acid in an aequous solution containing salts in a high concentration; (b) providing the substrate in the form of powdered material; (c) providing a water-miscible cyclic diether; (d) dispersing the substrate of step (b) in the water-miscible cyclic diether of step (c) to form a suspension of said substrate; and (e) mixing the aequous solution of step (a) with the suspension of step (d). Preferably, the water-miscible cyclic diether is dioxane.

Preferably, the composition of step (e) contains 1 to 50 per cent volume by volume of the water-miscible cyclic diether. More preferred, the composition of step (e) contains 2 to 35 per cent volume by volume of the water-miscible cyclic diether. Even more preferred, the composition of step (e) contains 3 to 30 per cent volume by volume of the water-miscible cyclic diether. Even more preferred, the composition of step (e) contains about 4 per cent volume by volume of the water-miscible cyclic diether. Even more preferred, the composition of step (e) contains about 15 per cent volume by volume of the water-miscible cyclic diether. Even more preferred, the composition of step (e) contains about 25 per cent volume by volume of the water-miscible cyclic diether.

Preferably, the salts in the composition of step (e) are chaotropic salts in concentrations of 1 to 8 M. More preferred, said chaotropic salts are selected from the group consisting of sodium perchlorate, guanidine hydrochloride, guanidine thiocyanate, guanidine isothiocyanate, and sodium iodide.

Also preferred, the salts in the composition of step (e) are in concentrations of 1 to 10 M and said salts are selected from the group consisting of lithium chloride, sodium chloride, potassium chloride, sodium acetate, urea, and mixtures thereof.

Preferably, the substrate comprises a powdered porous or non-porous mineral substrate selected from the group consisting of silica gel, glass, quartz, and zeolites. Also preferred, the substrate comprises a powdered porous or non-porous mineral substrate selected from the group consisting of metal oxides, and/ or metal mixed oxides, alumina, titania, zirconia, and materials predominantly consisting of glass. It is also preferred that the substrate comprises magnetically attractable particles. More preferred, the magnetically attractable particles are coated with a mineral substrate selected from the group consisting of silica gel, glass, quartz, and zeolites. Even more preferred, the substrate comprises magnetically attractable particles coated with glass.

Further useful is a suspension containing a substrate in the form of powdered material dispersed in a water-miscible cyclic diether. Preferably, the water-miscible cyclic diether is dioxane.

Preferably, the substrate comprises a powdered porous or non-porous mineral substrate selected from the group consisting of silica gel, glass, quartz, and zeolites. Also preferred, the substrate comprises a powdered porous or non-porous mineral substrate selected from the group consisting of metal oxides, and/ or metal mixed oxides, alumina, titania, zirconia, and materials predominantly consisting of glass. It is also preferred that the substrate comprises magnetically attractable particles. More preferred, the magnetically attractable particles are coated with a mineral substrate selected from the group consisting of silica gel, glass, quartz, and zeolites. Even more preferred, the substrate comprises magnetically attractable particles coated with glass.

Further useful is the use of a water-miscible cyclic diether, for performing the methods according to the invention described herein. Preferably, the cyclic diether is dioxane.

Also useful is the use of a water-miscible cyclic diether, for preparing a suspension by way of dispersing a substrate in said water-miscible cyclic diether to form a suspension of said substrate. Preferably, the cyclic diether is dioxane.

Preferably, the substrate comprises a powdered porous or non-porous mineral substrate selected from the group consisting of silica gel, glass, quartz, and zeolites. Also preferred, the substrate comprises a powdered porous or non-porous mineral substrate selected from the group consisting of metal oxides, and/ or metal mixed oxides, alumina, titania, zirconia, and materials predominantly consisting of glass. It is also preferred that the substrate comprises magnetically attractable particles. More preferred, the magnetically attractable particles are coated with a mineral substrate selected from the group consisting of silica gel, glass, quartz, and zeolites. Even more preferred, the substrate comprises magnetically attractable particles coated with glass.

Further useful is the use of a suspension in dioxane described above for performing a method according to the invention as described herein.

Also useful is a kit of parts, containing (a) a concentrated stock solution of a buffer salt and a chaotropic salt selected from the group consisting of sodium perchlorate, guanidine hydrochloride, guanidine thiocyanate, guanidine isothiocyanate, and sodium iodide; (b) dioxane; (c) buffer solutions; and (d) chromatographic and filtering material.

Further useful is a kit of parts, containing (a) a concentrated stock solution of a buffer salt and a salt selected from the group consisting of lithium chloride, sodium chloride, potassium chloride, sodium acetate, urea, and mixtures thereof; (b) dioxane; (c) buffer solutions; and (d) chromatographic and filtering material.

Also useful is a kit of parts, containing (a) a concentrated stock solution of a buffer salt and a chaotropic salt selected from the group consisting of sodium perchlorate, guanidine hydrochloride, guanidine thiocyanate, guanidine isothiocyanate, and sodium iodide; (b) a suspension in dioxane described above; (c) buffer solutions.

Further useful is a kit of parts, containing (a) a concentrated stock solution of a buffer salt and a salt selected from the group consisting of lithium chloride, sodium chloride, potassium chloride, sodium acetate, urea, and mixtures thereof; (b) a suspension in dioxane described above; (c) buffer solutions.

The following examples, references, and figures are provided to aid the understanding of the present invention, the true scope of which is set forth in the appended claims.

## Description of the Figures

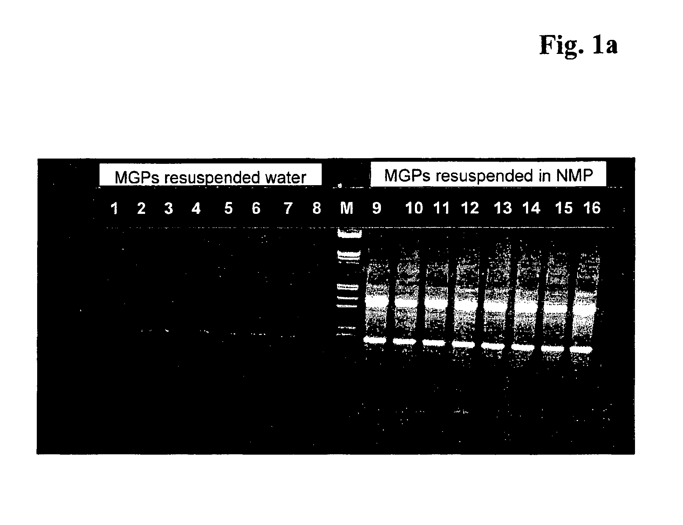

Figure 1a      RNA was isolated from $10^6$ HeLa cells in 8-fold replicates using the respective protocol and kit of the MagNA Pure System (Roche Diagnostics GmbH, Mannheim). For these isolations, the MGPs (magnetic glass particles) were resuspended either in water or in N-methyl-2-pyrrolidone (NMP). The eluates with purified RNA were then analysed on an agarose gel. The bands in lanes 1 - 8, i.e. RNA preparations using MGPs resuspended in water, are very weak and can hardly be reproduced. M indicates a size marker. Lanes 9 - 16 show RNA bands from preparations using MGPs resuspended NMP.

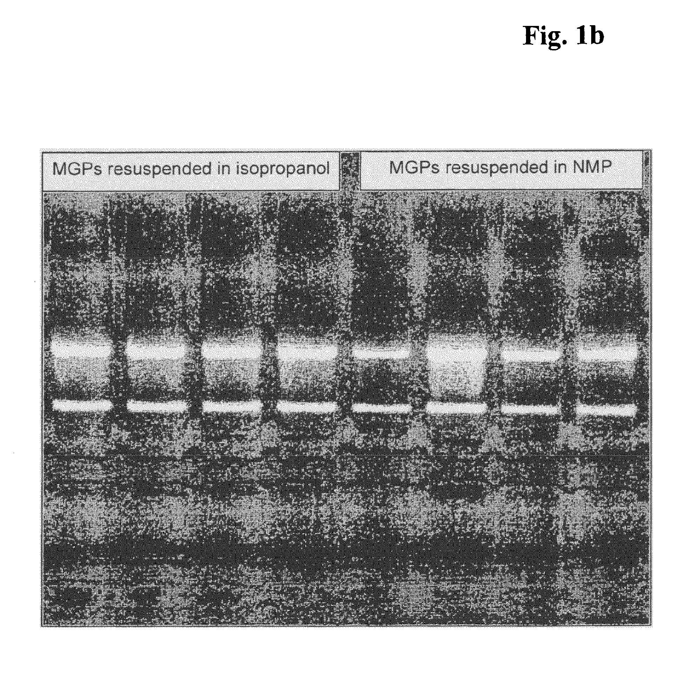

**Figure 1b**     RNA was isolated from $10^6$ HeLa cells in 4-fold replicates using the respective protocol and kit of the MagNA Pure System (Roche Diagnostics GmbH, Mannheim; catalogue no. 3186229). For these isolations, the MGPs (magnetic glass particles) were resuspended either in isopropyl alcohol (isopropanol) or in N-methyl-2-pyrrolidone (NMP). The eluates with purified RNA were then analysed on an agarose gel.

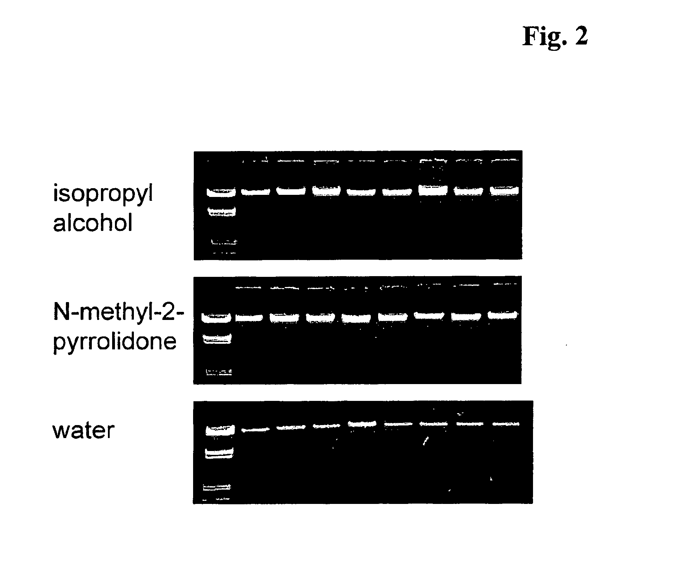

**Figure 2**     DNA was isolated from 1 ml of blood in 8-fold replicates using the respective protocol and kit of the MagNA Pure System (Roche Diagnostics GmbH, Mannheim; catalogue no. 3186229). For these isolations, the MGPs (magnetic glass particles) were resuspended either in Isopropanol or in N-methyl-2-pyrrolidone or in water. The eluates with purified DNA were then analysed on an agarose gel.

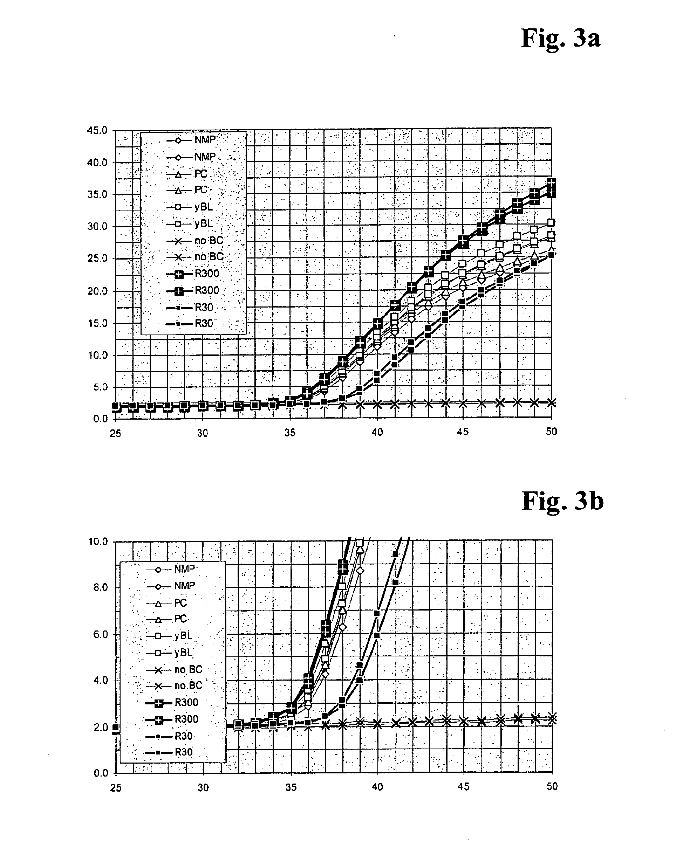

**Figures 3a, 3b**     Fluorescence signal during TaqMan PCR. The x-axis indicates the number of PCR cycles, the y axis the fluorescence signal as measured by the detector in [mV]. The "R300" curves reflect the signals obtained with 10,000 copies of positive control target RNA in purified water. The "R30" curves reflect the signals obtained with 1,000 copies of positive control target RNA in purified water. Additional curves are given for "NMP" (1-methyl-2-pyrrolidone), "PC" (propylene carbonate), "yBL" (gamma-butyrolactone), and "no BC" (without the addition of a binding conditioner).

## Example 1

### DNA isolation using glass fiber

1,000 μl of whole blood from a healthy donor was incubated with 100 μl Proteinase K solution (Roche Id.No. 745723; 90 mg dissolved in 4.5 ml water) and 1,200 μl chaotropic binding buffer (6 M Guanidinium-HCl, 10 mM TrisHCl, 20% Triton X100 pH 4.4) at 70°C for 10 minutes.

After adding 500 μl of (a) isopropanol, (b) acetonitrile, (c) dimethylsulfoxide or (d) methylethylketone, the lysate was transferred to a glass fiber filter tube (filter tube taken from the kit of Macherey und Nagel Cat.No. 740 954.20). After centrifugation for 3 minutes at 1,900 xg the flowthrough was discarded and the filter tube was placed on a collection tube. 2,000 μl of a high ionic inhibitor removal buffer (5M Guanidinuim-HCl, 20 mM Tris, 60% Ethanol, pH 6.6) was pipetted on the glass fiber filter and centrifuged for 1 minute at 3,000 xg. Followed by two washing steps with 2,000 μl wash buffers (20 mM NaCl, 2 mM TrisHCl, 80% ethanol, pH 7.5) and centrifugation for 5 minutes at 3,000 xg. The flowthrough was discarded. A new colletion tube was used. The elution of the DNA was done with 300 μl of 70°C hot Tris buffer (10mM, pH 8.5). After a incubation time of 5 minutes the tube was centrifuged for 5 minutes at 3,000 xg.

### Analysis of the isolated DNA

The DNA yields were calculated from the OD260nm measurement using a standard photometer. The purity was assessed by calculating the ratio OD260/280nm. The results (n= 2) are depicted in Table 1.

**Table 1**

| Isolation of DNA from 1,000 μl whole blood | | |
|---|---|---|
| adsorbtion to the substrate in the presence of | Yield (measured by determining OD at 260 nm) | Purity (ratio 260/280 nm) |
| (a) isopropanol | 24.1 μg/ml blood | 1.89 |
| (b) acetonitrile | 22.2 μg/ml blood | 1.88 |
| (c) dimethylsufoxide | 23.5 μg/ml blood | 1.90 |
| (d) methylethylketone | 33.5 μg/ml blood | 1.81 |

### Example 2

**RNA isolation on the MagNA Pure LC instrument**

$10^6$ Hela cells (in a volume of 200 $\mu$l) were directly transferred to the sample cartridge of the MagNA Pure LC instrument (Roche Diagnostics GmbH, Mannheim). The respective protocol was chosen from the software, the necessary plastic disposables and kit reagents were loaded onto the workstation, and the automated RNA isolation was started. The MagNA Pure LC instrument then automatically performed all isolation and purification steps like cell lysis with a special Lysis/Binding Buffer, enzymatic protein digest with Proteinase K, enzymatic DNA digest with DNase I, binding of RNA to Magnetic Glass Particles (MGPs), several washing steps to remove unbound substances and impurities, elution of the pure RNA in a special elution buffer and finally the transfer of the eluate to a cooled storage cartridge.

When non-acidic organic compounds such as N-methyl-2-pyrrolidone were to be analysed with respect to their performance in the nucleic acid isolation procedure, dry MGPs were suspended in a suitable volume of the respective non-acidic organic compound and the suspension was used in the MagNA Pure LC instrument together with all other reagents of the MagNA Pure kit (Roche Diagnostics GmbH, Mannheim).

Compared to water, the RNA yield was found to be higher when the suspension of the magnetic glass particles was prepared using N-methyl-2-pyrrolidone. Compared to isopropyl alcohol, the RNA yield was found to be equal or higher when the suspension of the magnetic glass particles was prepared using N-methyl-2-pyrrolidone.

**Table 2**

| Isolation of RNA from $10^6$ HeLa cells (see also Figure 1a, Figure 1b) | | |
|---|---|---|
| **MGPs suspension in** | **Yield (measured by determining OD at 260 nm)** | **Purity (ratio 260/280 nm)** |
| (i) dest. water | 1.97 $\mu$g/$10^6$ cells | 1.90 |
| (ii) N-methyl-2-pyrrolidone | 15.90 $\mu$g/$10^6$ cells | 2.02 |
| (iii) N-methyl-2-pyrrolidone | 14.53 $\mu$g/$10^6$ cells | 2.00 |
| (iv) isopropyl alcohol | 14.31 $\mu$g/$10^6$ cells | 2.00 |

Results are also shown on Figures 1a (i, ii) and 1b (iii, iv).

### Example 3

**DNA isolation on the MagNA Pure LC instrument**

Human blood (1 ml) was directly transferred to the sample cartridge of the MagNA Pure LC instrument (Roche Diagnostics GmbH, Mannheim). The respective protocol was chosen from the software, the necessary plastic disposables and kit reagents were loaded onto the workstation, and the automated DNA isolation was started. The MagNA Pure LC instrument then automatically performed all isolation and purification steps like cell lysis with a special Lysis/Binding Buffer, enzymatic protein digest with Proteinase K, binding of DNA to Magnetic Glass Particles (MGPs), several washing steps to remove unbound substances and impurities, elution of the pure DNA in a special elution buffer and finally the transfer of the eluate to a cooled storage cartridge.

When non-acidic organic compounds such as N-methyl-2-pyrrolidone were to be analysed with respect to their performance in the nucleic acid isolation procedure, dry MGPs were suspended in a suitable volume of the respective non-acidic organic compound and the suspension was used in the MagNA Pure LC instrument together with all other reagents of the MagNA Pure kit (Roche Diagnostics GmbH, Mannheim).

**Analysis of the isolated DNA**

The integrity of the isolated DNA was checked on a 1% agarose gel, stained with ethidium bromide, together with molecular weight marker III (Roche Diagnostics GmbH, Mannheim). The DNA yields were calculated from the OD260nm measurement using a standard photometer. The purity was assessed by calculating the ratio OD260/280nm. To secure that the DNA isolated using the MagNA Pure protocols and N-methyl-2-pyrrolidone or other non-acidic organic compounds can be amplified, PCR on a LightCycler instrument was performed for all samples using e.g. the LightCycler Factor V Mutation Detection Kit or the LightCycler Her2neu DNA Quantification Kit (both Roche Diagnostics GmbH, Mannheim). Amplification was successful in all cases.

**Table 3**

| Isolation of DNA from 1,000 µl whole blood (see also Figure 2) | | |
|---|---|---|
| MGPs suspension in | Yield (measured by determining OD at 260 nm) | Purity (ratio 260/280 nm) |
| (i) isopropyl alcohol | 25.3 µg/ml blood | 1.70 |
| (ii) N-methyl-2-pyrrolidone | 26.6 µg/ml blood | 1.75 |
| (iii) water | 7.3 µg/ml blood | 1.69 |

The DNA yield with N-methyl-2-pyrrolidone was the same as with isopropyl alcohol, the yield with water was much lower. Figure 2 illustrates these results.

**Example 4**

**RNA isolation from a serum sample**

**Lysis and conditioning**

A volume of 200 µl serum from a healthy patient was mixed with 20 µl proteinase K solution (enzyme activity 6,000 U/ml, free of DNase and RNAse activity) and incubated for 5 min at 37°C. Subsequently, 600 µl of lysis buffer was mixed with the proteinase K-treated sample to result in a lysis solution. The lysis buffer contained the following compounds dissolved in water:

**Table 4**

| quantity | unit | substance | manufacturer | product number |
|---|---|---|---|---|
| 6.2 | mol/l | Guanidium-rhodanide | Fluka | 50981 |
| 0.04 | mol/l | Tris HCl pH 7.5 | Fluka | 93372 |
| 10 | g/l | Triton X100 | Fluka | 93426 |
| 0.02 | mol/l | 1,4-Dithio-DL-threit | Fluka | 43816 |
| 15.6 | mg/l | Poly-A | Amersham Biosciences | 27-4110-01 |

Subsequently, 380 µl of a binding conditioner was added to the lysis solution and mixed to result in an adsorption solution. 100% Gamma-butyrolactone (CAS 96-48-0), 100% propylene carbonate (CAS 108-32-7) or 100% 1-methyl-2-pyrrolidone (CAS872-50-4) were used as binding conditioners.

**Adsorbing to a solid phase**

A first 600 µl aliquot of the conditioned lysis solution was transferred to a commercially available spin column with a glass fleece as a solid phase. Preferably, High Pure Spin Filter tubes from the High Pure PCR Template Preparation Kit (Roche Diagnostics GmbH, Mannheim, Germany, Catalogue No. 1796828) were used as spin columns. The spin column with the first 600 µl aliquot was centrifuged at 4,300 x g for 1 min. The second aliquot was then transferred to the same spin column and the centrifugation step was repeated under the same conditions.

**Washing**

The column was washed three times, each time using 150 µl of washing buffer. The washing buffer contained the following compounds dissolved in water:

**Table 5**

| quantity | unit | substance | manufacturer | product number |
|---|---|---|---|---|
| 600 | g/l | Ethanol/Isopropanol= 19:1 | Fluka | 2848 |

(continued)

| quantity | unit | substance | manufacturer | product number |
|---|---|---|---|---|
| 0.66 | mmol/l | TrisHCl pH 7.5 | Fluka | 93372 |
| 10 | mg/l | Poly A | Amersham Biosciences | 27-4110-01 |

For the first two washing steps the aliquot of washing buffer was transferred to the spin column and the column was centrifuged at 4,300 x g for 1 min. The third washing step was altered in that the column was centrifuged at 13,200 x g for 3 min.

Instead of removing the washing buffer by means of centrifugation (third washing step, see above) the solid phase was alternatively dried at 65°C for 10 min.

**Elution**

For this step elution buffer was used which contained the following compound dissolved in water:

**Table 6**

| quantity | unit | substance | manufacturer | product number |
|---|---|---|---|---|
| 3.3 | mmol/L | TrisHCl pH 7.5 | Fluka | 93372 |

A volume of 150 $\mu$l elution buffer was transferred to the spin column and the column was centrifuged at 4,300 x g for 1 min. The eluate was collected for further analysis.

**Example 5**

**RNA analysis**

Serum samples spiked with 10,000 copies of a positive control target RNA (purified hepatitis C virus RNA) were processed as described in Example 4. The content of target RNA in the eluate was determined by means of TaqMan PCR using a Roche HCV detection kit.

For the calclulation of the recovery rate (= amount found after the process / amount before process) standards were run with the detection procedure. Figures 3a and 3b show the "R300" curve corresponding to 10,000 copies (i.e. 300% recovery rate) and the "R30" curve corresponding to 1,000 copies (i.e. 30% recovery rate).

Figures 3a and 3b illustrate the outcome of a typical experiment. While a control experiment without binding conditioner ("noBC") leads to very low recovery rates (no target found), adding a binding conditioner ("NMP": N-methyl-2-pyrrolidone, "PC": propylen cabronate or "gBL": gamma-butyrolactone) leads to a recovery rate of more than 50% for the control RNA.

Sample preparation procedures which leave impurities in the eluate may impair signal formation during the TaqMan PCR process. Signal formation was therefore monitored to estimate the quality/purity of the RNA preparation. The value of the fluorescence signal after the last PCR cycle was taken as a measure. As a reference known amounts of clean positive control RNA (the same as spiked to the serum) in pure water was used. Figures 3a and 3b illustrate the outcome of a typical experiment. While the formation of fluorescence signal was negligible in the preparations without binding conditioner (mainly due to the missing recovery), adding binding conditioner leads to a improved signal formation which is comparable to the signal formation found with a pure target.

**Example 6**

**Sample processing time**

Samples received from a hospital, whereby the samples had enhanced values of triglycerides, were processed according to to following protocol:

A volume of 750 $\mu$l Serum was incubated for 5 min with 75 $\mu$l proteinase K solution (enzyme activity 6,000 U/ml, free of DNase and RNAse activity) at 37°C. Afterwards, a volume of 1,405 $\mu$l lysis buffer (according to Table 4) was added and mixed. Subsequently, 880 $\mu$l gamma-butyrolactone or, alternatively, 880 $\mu$l ethanol 96% were added and mixed, resulting in two different types of adsorption solution.

Each adsorption solution was processed at a constant pressure of +1 bar through a column device containing a glass-fiber-fleece (used from a Roche "High Pure" kit) in a diameter of 5 mm and a thickness of 1 mm. The time for passing of the whole volume through the device (also referred to as "binding time") was measured. The results are summarized in Table 7.

**Table 7**

| Sample-ID | triglyceride content [mmol/Liter] | Binding-Time using gamma-butyrolactone [sec] | Binding-Time using ethanol [sec] |
|---|---|---|---|
| 3C | 1.78 * | 97 | 91 |
| 1C | 3.73** | 92 | 128 |
| 9C | 5.73** | 92 | 103 |
| 2C | 7.18** | 94 | 148 |
| * regarded as normal, ** elevated triglcerides value | | | |

### List of References

Abramson, R.D., and Myers, T.W., Curr. Opin. Biotechnol. 4 (1993) 41-47
Alderton, R.P., et al., Anal. Biochem. 201 (1992) 166-169
Ausubel et al., Current Protocols in Molecular Biology, J. Wiley and Sons, NY, 1987
Barany, F., PCR Methods and Applic. 1 (1991) 5-16
Barany, F., Proc. Natl. Acad. Sci. USA 88 (1991) 189-193
Boom, R., et al., J. Clin. Microbiol. 28 (1990) 495-503
DE 37 24 442
EP 0 439 182 A2
EP 0 658 164
Guatelli, J.C., et al., Proc. Natl. Acad. Sci. USA 87 (1990) 1874-1878
Jakobi, R., et al., Anal. Biochem. 175 (1988) 196-201
Kwoh, D.Y., et al., Proc. Natl. Acad. Sci. USA 86 (1989) 1173-1177
Marko, M.A., et al., Anal. Biochem. 121 (1982) 382-387
Sambrook, Fritsch & Maniatis, Molecular Cloning, A Laboratory Manual, 3rd edition, CSHL Press, 2001
US 4,683,195
US 5,130,238
US 5,210,015
US 5,487,972
US 5,804,375
Vogelstein, B., and Gillespie, D., Proc. Natl. Acad. Sci. USA 76 (1979) 615-619
Whelen, A.C., and Persing, D.H., Annu. Rev. Microbiol. 50 (1996
WO 01/37291
WO 90/01069
WO 91/00212
WO 92/02638
WO 92/08800
WO 99/16781
Wu, D.Y., and Wallace, R.B., Genomics 4 (1989) 560-569
Yamada, O., et al., J. Virol. Methods 27 (1990) 203-209

### Claims

1. A method for the purification of a nucleic acid, comprising the steps of:

   (a) adsorbing on a porous or non-porous mineral substrate selected from the group consisting of silica gel, glass fibers, quartz fibers, zeolites, and magnetically attractable particles coated with glass the nucleic acid sequence-independently from a composition containing

(i) an aqueous buffer,
(ii) salts in a concentration equal to or greater than 1 M,
(iii) a water-miscible non-acidic organic compound comprising a functional group of Formula I,

$$W \equiv Z \qquad \text{(Formula I)}$$

whereby W is a carbon atom or a sulfur atom and Z is an oxygen atom or a nitrogen atom,
whereby the functional group is selected from the group consisting of an oxo group, a sulfoxo group, a cyano group, and a carbonyl group of a carbamoyl function or an amide but not belonging to a carboxy function, and
the water-miscible non-acidic organic compound is selected from the group consisting of acetylacetone, dimethylsulfoxide, diethylketone, methylethylketone, methylpropylketone, isobutylmethylketone, gamma-butyrolactone, gamma-valerolactone, propylene carbonate, and N-methyl-2-pyrrolidone, and
whereby composition of step (a) contains 1 to 50 per cent volume by volume of the water-miscible non-acidic organic compound, and the water-miscible non-acidic organic compound is dissolved to form a homogeneous liquid phase,
and
(iv) the nucleic acid;

(b) optionally washing with a washing solution the substrate with the adsorbed nucleic acid; followed by
(c) contacting the substrate with the adsorbed nucleic acid with a solution containing salts in a lower concentration compared to the composition of step (a), thereby desorbing the nucleic acid from the substrate; and
(d) separating the solution with the desorbed nucleic acid from the substrate, thereby purifying the nucleic acid; and optionally
(e) precipitating the desorbed nucleic acid from the solution of step (d) and isolating the precipitated nucleic acid, thereby further purifying the nucleic acid.

2. The method according to claim 1, **characterized in that**
the composition of step (a) contains 3 to 30 per cent volume by volume of the water-miscible non-acidic organic compound.

3. The method according to any of the claims 1 and 2, **characterized in that**
the salts in the composition of step (a) are chaotropic salts in concentrations of 1 to 8 M and said chaotropic salts are selected from the group consisting of sodium perchlorate, guanidine hydrochloride, guanidine thiocyanate, guanidine isothiocyanate, and sodium iodide.

4. The method according to any of the claims 1 and 2, **characterized in that** the salts in the composition of step (a) are in concentrations of 1 to 10 M and said salts are selected from the group consisting of lithium chloride, sodium chloride, potassium chloride, sodium acetate, urea, and mixtures thereof.

5. The method according to any of the claims 1 to 4, **characterized in that**
the washing solution of step (b) contains the water-miscible non-acidic organic compound.

6. The method of claim 5, **characterized in that**
the washing solution of step (b) contains between 1 and 100 per cent volume by volume of the water-miscible non-acidic organic compound.

7. The method according to claim 1, **characterized in that** the water-miscible non-acidic organic compound is gamma-butyrolactone and the mineral substrate consists of glass fibers.

8. The method according to claim 1, **characterized in that** the nucleic acid is RNA and the water-miscible non-acidic organic compound is N-methyl-2-pyrrolidone.

**Patentansprüche**

1.  Verfahren zur Aufreinigung einer Nukleinsäure, das folgende Schritte umfasst:

    (a) sequenzunabhängiges Adsorbieren der Nukleinsäure an einem porösen oder nicht porösen Mineralsubstrat aus der Gruppe bestehend aus Kieselgel, Glasfasern, Quarzfasern, Zeolithen und magnetisch anziehbaren Partikel, die mit Glas beschichtet sind, aus einer Zusammensetzung, enthaltend

    (i) einen wässrigen Puffer,
    (ii) Salze in einer Konzentration größer gleich 1 M,
    (iii) eine mit Wasser mischbare nicht acide organische Verbindung mit einer funktionellen Gruppe der Formel I,

    $$W \equiv Z \qquad\qquad (Formel\ I)$$

    wobei W für ein Kohlenstoffatom oder ein Schwefelatom steht und Z für ein Sauerstoffatom oder ein Stickstoffatom steht,
    wobei die funktionelle Gruppe aus der Gruppe bestehend aus einer Oxogruppe, einer Sulfoxogruppe, einer Cyanogruppe und einer Carbonylgruppe einer Carbamoylfunktion oder eines Amids, die aber nicht zu einer Carboxyfunktion gehört, ausgewählt ist und
    die mit Wasser mischbare nicht acide organische Verbindung aus der Gruppe bestehend aus Acetylaceton, Dimethylsulfoxid, Diethylketon, Methylethylketon, Methylpropylketon, Isobutylmethylketon, gamma-Butyrolacton, gamma-Valerolacton, Propylencarbonat und N-Methyl-2-pyrrolidon ausgewählt ist und
    wobei die Zusammensetzung von Schritt (a) 1 bis 50 Volumenprozent der mit Wasser mischbaren nicht aciden organischen Verbindung enthält und die mit Wasser mischbare nicht acide organische Verbindung zur Bildung einer homogenen flüssigen Phase gelöst wird,
    und
    (iv) die Nukleinsäure;

    (b) gegebenenfalls Waschen des Substrats mit der adsorbierten Nukleinsäure mit einer Waschlösung; gefolgt von
    (c) Inkontaktbringen des Substrats mit der adsorbierten Nukleinsäure mit einer Lösung, die Salze in niedrigerer Konzentration als die Zusammensetzung von Schritt (a) enthält, wodurch die Nukleinsäure von dem Substrat desorbiert wird; und
    (d) Abtrennen der Lösung mit der desorbierten Nukleinsäure von dem Substrat, wodurch die Nukleinsäure gereinigt wird; und gegebenenfalls
    (e) Ausfällen der desorbierten Nukleinsäure aus der Lösung von Schritt (d) und Isolieren der ausgefallenen Nukleinsäure, wodurch die Nukleinsäure weiter gereinigt wird.

2.  Verfahren nach Anspruch 1, **dadurch gekennzeichnet, dass**
    die Zusammensetzung von Schritt (a) 3 bis 30 Volumenprozent der mit Wasser mischbaren nicht aciden organischen Verbindung enthält.

3.  Verfahren nach einem der Ansprüche 1 und 2, **dadurch gekennzeichnet, dass**
    es sich bei den Salzen in der Zusammensetzung von Schritt (a) um chaotrope Salze in Konzentrationen von 1 bis 8 M handelt und die chaotropen Salze aus der Gruppe bestehend aus Natriumperchlorat, Guanidinhydrochlorid, Guanidinthiocyanat, Guanidinisothiocyanat und Natriumiodid ausgewählt sind.

4.  Verfahren nach einem der Ansprüche 1 und 2, **dadurch gekennzeichnet, dass**
    die Salze in der Zusammensetzung von Schritt (a) in Konzentrationen von 1 bis 10 M vorliegen und die Salze aus der Gruppe bestehend aus Lithiumchlorid, Natriumchlorid, Kaliumchlorid, Natriumacetat, Harnstoff und Mischungen davon ausgewählt sind.

5.  Verfahren nach einem der Ansprüche 1 bis 4, **dadurch gekennzeichnet, dass**
    die Waschlösung von Schritt (b) die mit Wasser mischbare nicht acide organische Verbindung enthält.

**6.** Verfahren nach Anspruch 5, **dadurch gekennzeichnet, dass**
die Waschlösung von Schritt (b) zwischen 1 und 100 Volumenprozent der mit Wasser mischbaren nicht aciden organischen Verbindung enthält.

**7.** Verfahren nach Anspruch 1, **dadurch gekennzeichnet, dass** es sich bei der mit Wasser mischbaren nicht aciden organischen Verbindung um gamma-Butyrolacton handelt und das Mineralsubstrat aus Glasfasern besteht.

**8.** Verfahren nach Anspruch 1, **dadurch gekennzeichnet, dass** es sich bei der Nukleinsäure um RNA handelt und es sich bei der mit Wasser mischbaren nicht aciden organischen Verbindung um N-Methyl-2-pyrrolidon handelt.

**Revendications**

**1.** Procédé pour la purification d'un acide nucléique, comprenant les étapes dans lesquelles

(a) on adsorbe sur un substrat minéral poreux ou non poreux choisi parmi le groupe constitué par du gel de silice, des fibres de verre, des fibres de quartz, des zéolithes et des particules enduites de verre, qui peuvent faire l'objet d'une attraction magnétique, la séquence d'acides nucléiques de manière indépendante à partir d'une composition contenant

(i) un tampon aqueux ;
(ii) des sels en une concentration égale ou supérieure à 1 M ;
(iii) un composé organique non acide miscible à l'eau comprenant un groupe fonctionnel répondant à la formule I

$$W \equiv Z \qquad \text{(Formule I)}$$

dans laquelle W représente un atome de carbone ou un atome de soufre et Z représente un atome d'oxygène ou un atome d'azote ;
le groupe fonctionnel étant choisi parmi le groupe constitué par un groupe oxo, un groupe sulfoxo, un groupe cyano et un groupe carbonyle d'une fonction carbamoyle ou un amide, mais qui ne fait pas partie d'une fonction carboxyle ; et
le composé organique non acide miscible à l'eau étant choisi parmi le groupe constitué par l'acétylacétone, le diméthylsulfoxyde, la diéthylcétone, la méthyléthylcétone, la méthylpropylcétone, l'isobutylméthylcétone la gammabutyrolactone, la gamma-valérolactone, le carbonate de propylène et la N-méthyl-2-pyrrolidone ; et
la composition de l'étape (a) contenant de 1 à 50 % en volume par volume du composé organique non acide miscible à l'eau, et le composé organique non acide miscible à l'eau étant dissous pour former une phase liquide homogène ;
et
(iv) l'acide nucléique ;

(b) de manière facultative, on lave avec une solution de lavage le substrat sur lequel est adsorbé l'acide nucléique ; et ensuite
(c) on met le substrat en contact avec l'acide nucléique adsorbé avec une solution contenant des sels en une concentration inférieure par rapport à celle de la composition de l'étape (a), pour ainsi obtenir une désorption de l'acide nucléique par rapport au substrat ; et
(d) on sépare la solution avec l'acide nucléique désorbé par rapport au substrat, pour ainsi purifier l'acide nucléique ; et de manière facultative ;
(e) on précipite l'acide nucléique désorbé à partir de la solution de l'étape (d) et on isole l'acide nucléique précipité, pour ainsi purifier davantage l'acide nucléique.

**2.** Procédé selon la revendication 1, **caractérisé en ce que** la composition de l'étape (a) contient de 3 à 30 % en volume par volume du composé organique non acide miscible à l'eau.

**3.** Procédé selon l'une quelconque des revendications 1 et 2, **caractérisé en ce que** les sels dans la composition de l'étape (a) sont des sels chaotropiques dans des concentrations de 1 à 8 M et lesdits sels chaotropiques sont choisis parmi le groupe constitué par le perchlorate de sodium, le chlorhydrate de guanidine, le thiocyanate de guanidine, l'isothiocyanate de guanidine et l'iodure de sodium.

4. Procédé selon l'une quelconque des revendications 1 et 2, **caractérisé en ce que** les sels dans la composition de l'étape (a) sont présents dans des concentrations de 1 à 10 M et lesdits sels sont choisis parmi le groupe constitué par le chlorure de lithium, le chlorure de sodium, le chlorure de potassium, l'acétate de sodium, l'urée et leurs mélanges.

5. Procédé selon l'une quelconque des revendications 1 à 4, **caractérisé en ce que** la solution de lavage de l'étape (b) contient le composé organique non acide miscible à l'eau.

6. Procédé selon la revendication 5, **caractérisé en ce que** la solution de lavage de l'étape (b) contient entre 1 et 100 % en volume par volume du composé organique non acide miscible à l'eau.

7. Procédé selon la revendication 1, **caractérisé en ce que** le composé organique non acide miscible à l'eau est la gamma-butyrolactone et le substrat minéral est constitué par des fibres de verre.

8. Procédé selon la revendication 1, **caractérisé en ce que** l'acide nucléique est de l'ARN et le composé organique non acide miscible à l'eau est la N-méthyl-2-pyrrolidone.

## Fig. 1a

**Fig. 1b**

MGPs resuspended in isopropanol   MGPs resuspended in NMP

**Fig. 2**

isopropyl
alcohol

N-methyl-2-
pyrrolidone

water

**Fig. 3a**

**Fig. 3b**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4683195 A
- DE 3724442
- WO 9100212 A
- WO 0137291 A
- EP 0658164 A
- EP 0512767 A
- WO 9001069 A
- EP 0439182 A2

- WO 9208800 A
- US 5130238 A
- WO 9202638 A
- US 5210015 A
- US 5804375 A
- US 5487972 A
- WO 9916781 A

**Non-patent literature cited in the description**

- **VOGELSTEIN, B. ; GILLESPIE, D.** *Proc. Natl. Acad. Sci. USA,* 1979, vol. 76, 615-619
- **BOOM, R. et al.** *J. Clin. Microbiol.,* 1990, vol. 28, 495-503
- **YAMADA, O. et al.** *J. Virol. Methods,* 1990, vol. 27, 203-209
- **MARKO, M.A. et al.** *Anal. Biochem.,* 1982, vol. 121, 382-387
- **JAKOBI, R. et al.** *Anal. Biochem.,* 1988, vol. 175, 196-201
- **ALDERTON, R.P. et al.** *Anal. Biochem.,* 1992, vol. 201, 166-169
- **SAMBROOK ; FRITSCH ; MANIATIS.** Molecular Cloning, A Laboratory Manual. CSHL Press, 2001
- **AUSUBEL et al.** Current Protocols in Molecular Biology. J. Wiley and Sons, 1987

- **WU, D.Y. ; WALLACE, R.B.** *Genomics,* 1989, vol. 4, 560-569
- **BARANY, F.** *Proc. Natl. Acad. Sci. USA,* 1991, vol. 88, 189-193
- **BARANY, F.** *PCR Methods and Applic.,* 1991, vol. 1, 5-16
- **KWOH, D.Y. et al.** *Proc. Natl. Acad. Sci. USA,* 1989, vol. 86, 1173-1177
- **GUATELLI, J.C. et al.** *Proc. Natl. Acad. Sci. USA,* 1990, vol. 87, 1874-1878
- **WHELEN, A.C. ; PERSING, D.H.** *Annu. Rev. Microbiol.,* 1996, vol. 50, 349-373
- **ABRAMSON, R.D. ; MYERS, T.W.** *Curr. Opin. Biotechnol.,* 1993, vol. 4, 41-47
- **BARANY, F.** *PCR Methods and Applic,* 1991, vol. 1, 5-16
- **WHELEN, A.C. ; PERSING, D.H.** *Annu. Rev. Microbiol.,* 1996, vol. 50